# EUROPÄISCHE PATENTSCHRIFT

(11) **EP 2 655 618 B1**
(45) Veröffentlichungstag und Bekanntmachung des Hinweises auf die Patenterteilung: **24.02.2016**
(21) Anmeldenummer: 11805485.7
(22) Anmeldetag: 20.12.2011
(51) Int. Cl.: C12N 15/10, C40B 40/08, C40B 50/06

(54) **VERFAHREN ZUR HERSTELLUNG VON LESERASTER-KORREKTEN FRAGMENT-BIBLIOTHEKEN**
METHOD FOR PRODUCING READING-FRAME-CORRECTED FRAGMENT LIBRARIES
PROCÉDÉ DE PRODUCTION DE BIBLIOTHÈQUES DE FRAGMENTS À CADRE DE LECTURE CORRECT

(30) Priorität: 24.12.2010 DE 102010056289
(43) Veröffentlichungstag der Anmeldung: 30.10.2013
(73) Patentinhaber: Geneart AG, 93053 Regensburg (DE)
(72) Erfinder: LISS, Michael, 93059 Regensburg (DE); KRANZ, Christian, 93049 Regensburg (DE)
(74) Vertreter: Weber, Birgit
(86) Internationale Anmeldenummer: PCT/EP2011/073373
(87) Internationale Veröffentlichungsnummer: WO 2012/084923

(56) Entgegenhaltungen:
- WO-A1-01/23602
- WO-A1-01/30998
- WO-A1-01/75158
- WO-A1-98/58080
- WO-A2-01/66798
- WO-A2-2006/024875
- WO-A2-2009/036379
- PRODROMOU ET AL: "DNA fragmentation-based combinatorial approaches to soluble protein expression, Part I. Generating DNA fragment libraries", DRUG DISCOVERY TODAY, ELSEVIER, RAHWAY, NJ, US, Bd. 12, Nr. 21-22, 23. Oktober 2007 (2007-10-23), Seiten 931-938, XP022338222, ISSN: 1359-6446, DOI: 10.1016/J.DRUDIS.2007.08.012
- SAVVA ET AL: "DNA fragmentation based combinatorial approaches to soluble protein expression, Part II: Library expression, screening and scale up", DRUG DISCOVERY TODAY, ELSEVIER, RAHWAY, NJ, US, Bd. 12, Nr. 21-22, 22. Oktober 2007 (2007-10-22), Seiten 939-947, XP022338223, ISSN: 1359-6446, DOI: 10.1016/J.DRUDIS.2007.08.016
- REICH STEFANIE ET AL: "Combinatorial Domain Hunting: An effective approach for the identification of soluble protein domains adaptable to high-throughput applications", PROTEIN SCIENCE, CAMBRIDGE UNIVERSITY PRESS, Bd. 15, Nr. 10, 1. Oktober 2006 (2006-10-01), Seiten 2356-2365, XP002599613, ISSN: 0961-8368, DOI: 10.1110/PS.062082606 [gefunden am 2009-01-01]

## Beschreibung

Die vorliegende Erfindung betrifft Verfahren zur Herstellung von Fragment-Bibliotheken zur Selektion funktioneller Polypeptidvarianten mit verbesserten Eigenschaften.

Bis zum heutigen Tage sind die Genome von mehr als 180 Organismen vollständig sequenziert. Bereits im Jahre 2001 war eine erste Version des humanen Genoms von Forschergruppen unabhängig voneinander publiziert worden (Lander et al., 2001, Nature 409:860-921; Venter et al., 2001, Science 291:1304-1351.1,2).
Neben dem menschlichen Erbgut sind die Genome zahlreicher Humanpathogene wie zum Beispiel Bacillus anthracis (Reed et al., 2002, Science 296:2028-2033), Helicobacter pylori (Tomb et al., 1997, Nature 388:539-547) oder Haemophilus influenzae (Fleischmann et al., 1995, Science 269:496-512.5) komplett entschlüsselt worden.

Es wird erwartet, dass sich zahlreiche pharmazeutisch relevante Targets unter den von den sequenzierten Genen kodierten Proteinen befinden. Um die bekannte Sequenzinformation nutzbar zu machen, werden biochemische Assays entwickelt, um die potentiellen Targets funktionell zu charakterisieren oder Ausgangsmaterial für die Selektion von Inhibitoren zu erzeugen. Überdies gewinnt die Strukturaufklärung von Proteinen eine immer wichtigere Rolle in der pharmazeutischen Industrie (Little et al., 2005, Genome Res. 15:1759-17666; Bentley et al., 2007, Drug Discov. Today 12:931-938).

Eine Grundvoraussetzung für die Charakterisierung des jeweiligen Proteins ist dessen quantitative Expression. Hierzu sollte das entsprechende Protein- oder Proteinderivat nicht nur in ausreichender Menge, sondern auch in löslicher Form produzierbar sein. Weitere Anforderungen an ein potentielles Drug Target sind dessen Funktionalität, Stabilität, die Möglichkeit es rein darzustellen und dessen Kristallisierbarkeit. Die dazu notwendige heterologe Expression der kodierenden Gene in einem geeigneten Expressionssystem sowie die anschließende Aufreinigung von richtig gefalteten rekombinanten Proteinen für strukturelle und funktionelle Analysen sind dabei die wohl kritischsten Schritte.

Die sogenannte "Target Data Base (TargetDB) for Structural Biology" (http://targetdb.pdb.org/) (Tabelle 1) liefert Informationen über den Status von Projekten, die der Strukturaufklärung von Proteinen dienen.

**Tabelle 1: TargetDB Status Statistics**

| Anzahl der Ziel ("Target")-Proteine, die weltweit von "Structural Genomics"-Zentren in der TargetDB hinterlegt wurden: 240.071 | | | | | |
|---|---|---|---|---|---|
| Status | Gesamtanzahl Targets | (%) relativ zu klonierten Targets | (%) relativ zu exprimierten Targets | (%) relativ zu gereinigten Targets | (%) relativ zu kristallisierten Targets |
| kloniert | 163639 | 100,0 | - | - | - |
| exprimiert | 117920 | 72,1 | 100,0 | - | - |
| löslich | 45629 | 27,9 | 38,7 | - | - |
| aufgereinigt | 41815 | 25,6 | 35,5 | 100,0 | - |
| kristallisiert | 14250 | 8,7 | 12,1 | 34,1 | 100,0 |
| Diffraktions-Qualität Kristalle | 7504 | 4,6 | 6,4 | 17,9 | 52,7 |
| Diffraktion | 6369 | 3,9 | 5,4 | 15,2 | 44,7 |
| NMR | 2131 | 1,3 | 1,8 | 5,1 | - |
| HSQC | 3848 | 2,4 | 3,3 | 9,2 | - |
| KristallStruktur | 5030 | 3,1 | 4,3 | 12,0 | 35,3 |
| NMR Struktur | 2030 | 1,2 | 1,7 | 4,9 | - |
| in ProteinDB | 7287 | 4,5 | 6,2 | 17,4 | 37 |
| Arbeit beendet | 28398 | - | - | - | - |
| Test Target | 109 | - | - | - | - |
| Andere | 10648 | - | - | - | - |

Die Statistik zeigt, dass sich bis Anfang 2010 knapp 28% der anvisierten Target-Proteine löslich exprimieren ließen. Weniger als 10% der Proteine konnten bisher kristallisiert werden.

In der Literatur sind verschiedene Ansätze beschrieben, um die Löslichkeit von größeren oder komplexeren Proteinen, welche nach Überexpression in E. coli häufig als unlösliche Aggregate in sogenannten inclusion bodies vorliegen, zu verbessern. So führte die Expression bei niedrigeren Temperaturen, die Verwendung unterschiedlich starker Promotoren oder die Fusion mit löslichen Tags, wie z.B. Hexa-Histidin (His6), Maltose-Bindeprotein (MBP), Glutathion-S-Transferase (GST) oder Thioredoxin (Trx) zu höheren Ausbeuten löslicher Zielproteine (Esposito und Chatterjee, 2006, Current Opinion in Biotechology 17:353-358). Auch auf DNA-Ebene kann bereits die Faltung und Löslichkeit von Proteinen beeinflusst werden. So wurde berichtet, dass die Kodon-Harmonisierung bzw. die Verwendung von seltenen Kodons an bestimmten Übergangsbereichen zwischen Proteindomänen zu einer verlangsamten Translation und somit zu einer verbesserten Faltung und Löslichkeit führen kann (US2008076161, Rosano und Ceccarelli, 2009, Microbial Cell Factories 8:41). Allerdings sind die oben beschriebenen Ansätze nur bedingt zuverlässig und oft nur für bestimmte Proteine geeignet.

Eine andere Möglichkeit, die genannten Schwierigkeiten mit der Löslichkeit oder Kristallisierbarkeit von Zielproteinen zu umgehen, beruht auf der Erzeugung von funktionellen Fragmenten dieser Proteine oder Polypeptide, welche durch bioinformatische Strukturvorhersagen auf Basis der zugrunde liegenden Aminosäuresequenz identifiziert wurden. Zu diesem Zweck wird eine verkürzte Variante des Zielproteins hergestellt, exprimiert und sodann auf Löslichkeit getestet. Oftmals lassen sich diese verkürzten Derivate signifikant besser löslich exprimieren und kristallisieren als die Volllänge-Proteine (Prodromou et al., 2007, Drug Discov. Today 12:931-938; Sawa et al., 2007, Drug Discov. Today 12:939-947).

Jedoch führen auch rationale Ansätze zur Strukturvorhersage von verkürzten Proteinsequenzen nicht immer zu Proteinvarianten mit den gewünschten Eigenschaften, da sich Veränderungen der Aminosäuresequenz kumulativ auf verschiedene Aspekte der Proteinstruktur auswirken können. Die derzeit verfügbaren bioinformatischen Programme können keine genauen Vorhersagen treffen, welche Modifikation oder Verkürzung die Löslichkeit des Proteins oder andere funktionelle Eigenschaften tatsächlich verbessern wird.

Aus diesem Grund werden Varianten- oder Fragment-Bibliotheken hergestellt, die verschiedene Längenvarianten des entsprechenden Proteins bzw. der für das Protein oder Polypeptid kodierenden Nukleinsäure-Ausgangssequenz enthalten. Hierfür werden Nukleinsäurekonstrukte, welche verkürzte (oder unter Umständen auch verlängerte oder mutierte) Varianten des Zielproteins kodieren, synthetisiert - z.B. durch de novo Synthese oder Amplifikation eines entsprechenden Teilbereiches der Nukleinsäure-Ausgangssequenz mittels eines Polymerase-Kettenreaktion (PCR)-basierten Verfahrens. So beschreibt beispielsweise WO 2009/036379 das Design, die *de novo* Synthese und Rekombination von synthetischen Antikörper-Bibliotheken, die Sequenzvarietäten und Längendiversitäten von natürlich vorkommenden menschlichen Sequenzen aufweist.

Die Verkürzungen können an nur einem oder auch an beiden Enden bzw. Termini erfolgen. Letzteres kann zielführend sein, falls ein natürlicher Terminus mit der Expression oder der Faltung des Proteins interferiert.

Um nicht jede Längenvariante innerhalb einer Fragment-Bibliothek separat herstellen zu müssen, werden beispielsweise Verfahren angewendet, die eine zeitgleiche Erzeugung verschiedener Fragmentlängen ermöglichen. Im Stand der Technik sind Methoden beschrieben, bei denen eine Nukleinsäure-Ausgangssequenz entweder enzymatisch verdaut (Campbell und Jackson, 1980, J. Biol. Chem. 255:3726-3735; Melgar und Goldthwait, 1968, J. Biol. Chem. 243:4409-4416; Price, 1972, J. Biol. Chem. 247:2895-2899; Anderson, 1981, Nucleic Acids Res. 9:3015-3027) oder physikalisch (z.B. durch Ultraschall) geschert wird (Hengen, 1997, Trends Biochem. Sci. 22:273-274; McKee et al., 1977, Biochemistry 16:4651-4654.14,15). Für die enzymatische Entfernung von einzelnen Nukleotiden an den Enden linearer Nukleinsäuresequenzen werden beispielsweise die Enyzme Exonuklease III oder Bal31 eingesetzt. (Rogers und Weiss, 1980, Meth. Enzymol. 65:201-211; Wie et al., 1983, J. Biol. Chem. 258:3506-13512). So beschreibt beispielsweise WO 98/58080 die Herstellung von Einzelstrang-Fragment-Bibliotheken mittels Bal31 Exonucleaseverdau.

Bei dem als "ITCHY" ("incremental truncation for the creation of hybrid enzymes") bezeichneten Verfahren (Ostermeir und Lutz, 2001, Methods in Molecular Biology 231:129-141) wird ein Zielgen jeweils an einem Ende durch Exonuklease III-Verdau trunkiert, während das andere Ende des Gens fixiert wird. Nachdem beide Enden des Gens separat trunkiert worden sind, werden die entstandenen Diversitäten z.B. durch Ligation der Fragmente kombiniert. In einem anderen Verfahren werden zufällige Verkürzungen mit Hilfe von Oligonukleotiden eingeführt, die in 3'-Richtung eines jeweils festgelegten Sequenzbereiches einen unbekannten variablen Sequenzbereich umfassen (WO0166798). Der variable Sequenzbereich der Oligonukleotide hybridisiert an geeignete Bereiche der Zielsequenz wodurch nach PCR-basierter Verlängerung der Oligonukleotide ("primer extension") unterschiedlich lange Fragmente der Nukleinsäure-Ausgangssequenz entstehen.

Die oben beschriebenen Verfahren zur Erzeugung von Fragment-Bibliotheken durch zufällige Verkürzung der Nukleinsäure-Ausgangssequenz haben den Nachteil, dass ca. zwei Drittel der resultierenden Fragmente Leseraster-Mutationen aufweisen. Weiterhin weisen die physikalisch gescherten oder unkontrolliert enzymatisch verkürzten Nukleinsäure-Fragmente individuelle 5'- und/oder 3'-Überhänge auf, welche nachfolgend enzymatisch weiter behandelt werden müssen (z.B. Auffüllen von 5'-Überhängen mit dem Klenow-Fragment der DNA Polymerase I oder Entfernen von 3'-Überhängen mit der 3'-5'-Exonuklease Aktivität der T4-Polymerase), um eine gleichzeitige "blunt end"-Klonierung aller Varianten in die jeweiligen Zielvektoren zu ermöglichen. Die ungerichtete "blunt end"-Klonierung führt zu einer weiteren Reduzierung von korrekten Konstrukten, so dass nur 1/3 x 1/2 = 1/6 aller Fragmente sowohl im korrekten Leserahmen, als auch in richtiger Orientierung im Expressionsvektor vorliegen (Prodromou et al., 2007, Drug Discov. Today 12:931-938; Savva et al., 2007, Drug Discov. Today 12:939-947).

Ein weiterer Nachteil der im Stand der Technik beschriebenen Verfahren ist die schwierige Kontrolle der den Verkürzungen zugrunde liegenden Parameter (Enzymmenge, Dauer der Inkubation, Leistung der Ultraschallsonde, etc.). Tendenziell ist es hier notwendig, mehrere Ansätze mit unterschiedlichen Parametern parallel zu bearbeiten, um einen Ansatz zu erhalten, der in etwa die geforderten Resultate erbringt.

Ein weiterer Nachteil der beschriebenen Verfahren ist, dass der Zentralbereich der Nukleinsäure-Ausgangssequenz, welche an den 5'- und 3'-Enden verkürzt werden soll, nur mit Aufwand frei wählbar ist. Da das Volllängenkonstrukt enzymatisch oder physikalisch gleichmäßig von beiden Seiten verkürzt wird, liegt der Zentralbereich zwangsläufig genau in der Mitte der Volllänge. Diese kann nur verschoben werden, indem ein Ende des Volllängenfragments zunächst verlängert wird, z.B. durch einen Anteil des Zielvektors oder mittels PCR-basierter Verfahren.

Limitierend ist auch die Tatsache, dass die 5'- und 3'-Verkürzungen graduell verlaufen. Es ist nicht möglich, Deletionen in größeren Schritten oder nur in definierten Blöcken (z.B. domänenweise) vorzunehmen. Dies erhöht die Diversität der resultierenden Fragment-Bibliothek und den Anteil unerwünschter Varianten und den damit verbundenen Screening Aufwand enorm. Zudem ist es wahrscheinlich, dass bestimmte Deletionen in der Fragment-Bibliothek häufiger vertreten sind, verursacht durch "hot spots" der Exonukleaseaktivität bzw. physikalisch fragile Positionen, die in der Scherung tendenziell leichter brechen.

Die der vorliegenden Erfindung zugrunde liegende Aufgabe besteht somit in der Bereitstellung von einem verbesserten Verfahren zur Herstellung von Fragment-Bibliotheken, wobei die Entstehung von Unsinn-Fragmenten vermieden wird. Insbesondere besteht die Aufgabe darin, Fragment-Bibliotheken herzustellen, die alle möglichen Kombinationen definierter Längenvarianten mit 5'- und 3'-Verkürzungen enthalten, wobei sowohl der zentrale Bereich als auch die exakten Längen der 5'- und 3'-Verkürzungen und deren Verteilung in der Fragment-Bibliothek frei wählbar sind.

Die Aufgabe der zugrunde liegenden Erfindung wird gelöst durch ein Verfahren zur Herstellung einer Fragment-Bibliothek umfassend mindestens zwei, bevorzugt mindestens vier Längenvarianten einer Nukleinsäure-Ausgangssequenz, gekennzeichnet dadurch dass
a) jede Längenvariante einen konstanten zentralen Bereich und jeweils einen 5'-und einen 3'- Bereich variabler Länge umfasst,
b) jede Längenvariante mindestens aus einem ersten Teilfragment und einem zweiten Teilfragment zusammengesetzt wird, wobei jedes erste Teilfragment mindestens einen definierten 5'-Teilbereich des konstanten zentralen Bereiches und jedes zweite Teilfragment mindestens einen definierten 3'-Teilbereich des konstanten zentralen Bereiches umfasst und die ersten und zweiten Teilfragmente keine Leserastermutationen gegenüber der Nukleinsäure-Ausgangssequenz aufweisen.
c) wobei alle ersten und zweiten Teilfragmente in einem Schritt so miteinander verbunden werden, dass die Längenvarianten alle möglichen Kombinationen aus ersten und zweiten Teilfragmenten aufweisen,
d) und optional keine Kombination von ersten Teilfragmenten miteinander oder zweiten Teilfragmenten miteinander erfolgen kann.

Weiterhin beschrieben wird eine Fragment-Bibliothek umfassend mindestens zwei, bevorzugt mindestens vier, sechs, acht, besonders bevorzugt mindestens 10, 12, 16, 20, 30, 40, 50, 60, 70, 80, 90 oder mindestens 100 Längenvarianten einer Nukleinsäure-Ausgangssequenz, gekennzeichnet dadurch dass
a) jede Längenvariante einen konstanten zentralen Bereich und jeweils einen 5'- und einen 3'- Bereich variabler Länge umfasst
b) jede Längenvariante mindestens aus einem ersten Teilfragment und einem zweiten Teilfragment zusammengesetzt ist, wobei jedes erste Teilfragment mindestens einen definierten 5'-Teilbereich des konstanten zentralen Bereiches und jedes zweite Teilfragment mindestens einen definierten 3'-Teilbereich des konstanten zentralen Bereiches umfasst

Die Fragment-Bibliothek ist weiterhin dadurch gekennzeichnet, dass alle ersten und zweiten Teilfragmente in einem Schritt so miteinander verbunden sind, dass die Längenvarianten alle möglichen Kombinationen aus ersten und zweiten Teilfragmenten aufweisen. In einer besonders bevorzugten Ausführungsform sind alle Längenvarianten zu annähernd gleichen Anteilen in der Fragment-Bibliothek vertreten.

Die Fragment-Bibliothek ist weiterhin dadurch gekennzeichnet, dass sie keine Unsinn-Fragmente enthält und die Längenvarianten nur aus eindeutig definierten ersten und zweiten Teilfragmenten zusammengesetzt sind und keine Leserastermutationen gegenüber der Nukleinsäure-Ausgangssequenz aufweisen.

Der konstante zentrale Bereich wird vorher definiert und ist bei jeder Längenvariante identisch. Dahingegen umfassen die Längenvarianten unterschiedliche 5'- und/oder 3'-Bereiche, deren Länge durch die jeweilige Länge der ersten und zweiten Teilfragmente festgelegt ist.

Die zugrunde liegende Nukleinsäure-Ausgangssequenz kann beispielsweise durch PCR-Amplifikation z.B. aus einer genomischen Sequenz, durch cDNA Synthese aus zellulären mRNAs oder durch Restriktionsverdau einer bestehenden Sequenz gewonnen werden. Alternativ kann die Nukleinsäure-Ausgangssequenz durch de novo-Synthese aus synthetischen Oligonukleotiden in Lösung oder durch Festphasensynthese generiert werden. Entsprechende Technologien und die zugrunde liegenden molekularbiologischen Verfahren zur Bereitstellung einer Nukleinsäure-Ausgangssequenz sind dem Fachmann bekannt und beispielsweise in WO03085094, US2003152984, WO9914318, WO02081490, WO0075368, WO0012123, US4652639 oder US5750380 beschrieben.

Die Nukleinsäure-Ausgangssequenz kann sowohl eine wildtypische Sequenz sein als auch eine modifizierte Sequenz oder aus wildtypischen und modifizierten Sequenzen zusammengesetzt sein. Die Modifikation kann beispielsweise darin bestehen, dass die Nukleinsäure-Ausgangssequenz partiell oder vollständig kodon-optimiert ist, was bedeutet, dass der Kodongebrauch für die Expression in einem Zielorganismus an das jeweilige Expressionssystem angepasst wird. Zusätzlich können Motive eingeführt werden, welche die Expression der Nukleinsäure modulieren. Ein Verfahren zur Modulation der Genexpression durch Änderung des Gehalts an intragenischen CpG-Dinukleotiden ist beispielsweise in WO2006015789 beschrieben. Es können sowohl Sequenzmotive, welche die Expression beeinträchtigen aus der Nukleinsäure-Ausgangssequenz entfernt werden, als auch Sequenzmotive, welche die Expression oder andere Funktionen fördern, in diese eingefügt werden. So können beispielsweise Computerprogramme und gängige Algorithmen verwendet werden, um unerwünschte Sequenzen wie z.B. RNA-Sekundärstrukturen, inhibitorische DNA- oder RNA-Elemente, kryptische Spleißstellen, unerwünschte Restriktionsschnittstellen etc. zu identifizieren und aus der Nukleinsäure-Ausgangssequenz zu entfernen. Falls es sich bei der Nukleinsäure-Ausgangssequenz um eine für ein Polypeptid oder Protein kodierende Sequenz handelt, können die Veränderungen basierend auf der Degeneration des genetischen Codes vorgenommen werden, ohne die Aminosäuresequenz zu verändern. Verfahren zur Sequenzoptimierung sind beispielsweise in WO04059556, WO06015789 oder in US114148 beschrieben.

Darüber hinaus kann für bestimmte Zwecke (z.B. Einführen zusätzlicher Mutationen) sowohl die Nukleinsäure-Ausgangssequenz als auch die abgeleiteten Teilfragmente oder Längenvarianten neben den gängigen DNA und RNA-Basen auch modifizierte Nukleobasen enthalten wie beispielsweise in Bergstrom, 2009, Curr. Protoc. Nucleic Acid Chem. 37:1.4.1-1.4.32 beschrieben.

Weiterhin kann die Nukleinsäure-Ausgangssequenz kodierend (für RNA, Protein, Polypeptid) oder nicht kodierend sein (z.B. eine Aptamersequenz umfassen). Sie kann für funktionelle RNA wie beispielsweise, rRNA, tRNA, snRNA, miRNA, siRNA, Satelliten-RNA, ein Ribozym oder Decoy kodieren.

Schließlich kann die Nukleinsäure-Ausgangssequenz aus mehreren Nukleinsäuren zusammengesetzt sein oder untranslatierte regulatorische Sequenzen umfassen, wie z.B. Promotoren, Transkriptions-, oder Translationssignale, Enhancer- oder Silencer-Sequenzen, Introns, ribosomale Bindestellen oder AT-reiche inhibitorische Elemente. Die untranslatierten Sequenzen können aber auch nicht regulatorische Funktionen wahrnehmen und beispielsweise als Platzhaltersequenzen dienen.

In einer bevorzugten Ausführungsform kodiert zumindest ein Teil der Nukleinsäure-Ausgangssequenz für eine Aminosäuresequenz, wobei die Aminosäuresequenz durch mindestens einen zentralen Bereich und einen amino-terminalen und/oder einen carboxyl-terminalen Bereich definiert ist und jede Längenvariante mindestens einen konstanten zentralen Bereich und jeweils einen amino-terminalen und/oder carboxyl-terminalen Bereich variabler Länge kodiert. In besonderen Fällen kann eine Längenvariante z.B. auch nur den konstanten zentralen Bereich und einen amino-terminalen Bereich oder nur den konstanten zentralen Bereich und einen carboxyl-terminalen Bereich aufweisen.

Bei der von der Nukleinsäure-Ausgangssequenz kodierten Aminosäuresequenz kann es sich um ein Protein, Polypeptid oder Polymer, einzelne oder kombinierte Domänen oder Fragmente davon sowie Derivate der genannten Aminosäuresequenzen oder um ein Fusionsprotein aus homologen oder heterologen Fusionsanteilen handeln.

Die Aminosäuresequenz kann darüber hinaus sowohl wildtypische, als auch rekombinante, chimäre bzw. artifizielle Sequenzbereiche umfassen. Des Weiteren kann die Aminosäuresequenz neben den natürlichen Aminosäuren auch unnatürliche Aminosäuren enthalten, welche zur Untersuchung von bestimmten physikalischen, chemischen oder biologischen Eigenschaften von Proteinen vorteilhaft sein können. In Bakterien können beispielsweise Stopp-Kodons für den Einbau unnatürlicher Aminosäuren unter Verwendung neuer oder modifizierter Enzyme "umprogrammiert" werden (Wang et al., 2001, Science 292:498-500). Die Erweiterung des genetischen Codes durch den Einbau unnatürlicher Aminosäuren und deren Verwendung ist z.B. für andere in vivo Expressionssysteme in WO04085463, WO10114615, WO09151491, WO09075847, WO08073184, WO08030612 und US2008254540 oder für die zellfreie Expression in WO08066583 beschrieben. Alternativ können Aminosäuren in den translatierten Fragmenten auch nachträglich (z.B. chemisch) modifiziert werden.

Die Fragment-Bibliothek kann mindestens zwei, bevorzugt mindestens vier, sechs, acht, 10, 20, 50, 100, 250, 500, 1000, 10.000, 25.000, 50.000, mindestens 100.000 oder mindestens 1.000.000 verschiedene Längenvarianten umfassen. Die gewünschte Diversität der Fragment-Bibliothek kann exakt bestimmt werden und hängt im Wesentlichen von der Länge der Nukleinsäure-Ausgangssequenz und der Länge des konstanten zentralen Bereiches sowie von der Fragestellung ab, wobei bei größeren Nukleinsäure-Ausgangssequenzen die Verkürzung nicht notwendigerweise aminosäureweise sondern auch in größeren Schritten, z.B. domänenweise erfolgen kann, um den Screening Aufwand gering zu halten. Es kann auch zunächst eine "Grobraster" Fragment-Bibliothek hergestellt werden, wobei die Verkürzungen in größeren Schritten (um mehr als drei Nukleotide bzw. mehr als eine Aminosäure) erfolgen. Nach Eingrenzung relevanter Bereiche kann aus diesen eine weitere "Feinraster" Fragment-Bibliothek mit aminosäureweisen Verkürzungen abgeleitet werden.

Dabei können die Längenvarianten der Fragment-Bibliothek gegenüber der Nukleinsäure-Ausgangssequenz
einen verkürzten 5'-Bereich oder
einen verkürzten 3'-Bereich oder
einen verkürzten 5'-Bereich und einen verkürzten 3'-Bereich aufweisen oder
einen verlängerten 5'-Bereich oder
einen verlängerten 3'-Bereich oder
einen verlängerten 5'-Bereich und einen verlängerten 3'-Bereich aufweisen, oder
einen verlängerten 5'-Bereich und einen verkürzten 3'-Bereich oder
einen verkürzten 5'-Bereich und einen verlängerten 3'-Bereich aufweisen oder
die Fragment-Bibliothek kann eine Teilmenge oder Kombination verschiedener oder aller möglichen dieser Längenvarianten umfassen.

Die Fragment-Bibliothek kann weiterhin Längenvarianten enthalten, die gegenüber der Nukleinsäure-Ausgangssequenz Insertionen oder interne Deletionen oder Punktmutationen innerhalb der ersten oder zweiten oder in den ersten und zweiten Teilfragmenten aufweisen.

Schließlich besteht die Möglichkeit, regulatorische Sequenzen, wie (z.B. Promoter/Enhancer- oder Terminatorsequenzen, untranslatierte Bereiche, Signalpeptidsequenzen, Introns, Restriktionsschnittstellen, Start- und Stoppkodons, Polyadenylierungs-Signalsequenzen etc.), die in der Nukleinsäure-Ausgangssequenz nicht enthalten sind, in die von der Nukleinsäure-Ausgangssequenz abgeleiteten Längenvarianten ein- oder anzufügen.

Die Fragment-Bibliothek ist ferner dadurch charakterisiert, dass sie mindestens zwei, bevorzugt mindestens vier, besonders bevorzugt mindestens 10 oder mindestens 100 Längenvarianten einer Nukleinsäure-Ausgangssequenz umfasst, welche einen konstanten zentralen Bereich und jeweils einen 5'-Bereich der durch X Nukleotide definiert ist und einen 3'- Bereich der durch Y Nukleotide definiert ist, umfasst, wobei X und Y ganze Zahlen sind und der zentrale Bereich in jeder Längenvariante identisch ist dadurch gekennzeichnet dass
a) der von der Nukleinsäure-Ausgangssequenz abgeleitete Teil des 5'-Bereiches im Vergleich zur Nukleinsäure-Ausgangssequenz um X - [m x 3] Nukleotide verkürzt ist, wobei m eine ganze Zahl und gleich oder kleiner X/3 ist oder
b) der von der Nukleinsäure-Ausgangssequenz abgeleitete Teil des 3'-Bereiches im Vergleich zur Nukleinsäure-Ausgangssequenz um Y - [n x 3] Nukleotide verkürzt ist, wobei n eine ganze Zahl und gleich oder kleiner Y/3 ist oder
c) der 5'-Bereich und der 3'-Bereich jeweils gemäß a) und b) verkürzt sind.

Weiterhin kann die Fragment-Bibliothek mindestens zwei, bevorzugt mindestens vier, sechs, acht, besonders bevorzugt mindestens 10, 12, 20, 30, 40, 50, 60, 70, 80, 90 oder mindestens 100 Längenvarianten einer für eine Aminosäuresequenz kodierenden Nukleinsäure-Ausgangssequenz umfassen, wobei die Aminosäuresequenz einen zentralen Bereich, einen amino-terminalen durch X Nukleotide kodierten Bereich und einen carboxyl-terminalen durch Y Nukleotide kodierten Bereich umfasst, wobei X und Y ganze Zahlen sind, dadurch gekennzeichnet dass der zentrale Bereich in jeder Längenvariante identisch ist und
a) die für den amino-terminalen Bereich kodierende Sequenz im Vergleich zur Nukleinsäure-Ausgangssequenz um X - [m x 3] Nukleotide verkürzt ist, wobei m eine ganze Zahl und gleich oder kleiner X/3 ist oder
b) die für den carboxyl-terminalen Bereich kodierende Sequenz im Vergleich zur Nukleinsäure-Ausgangssequenz um Y - [n x 3] Nukleotide verkürzt ist, wobei n eine ganze Zahl und gleich oder kleiner Y/3 ist oder
c) die für den amino-terminalen Bereich und den carboxyl-terminalen Bereich kodierenden Sequenzen jeweils gemäß a) und b) verkürzt sind

Beschrieben wird weiterhin ein Verfahren zur Herstellung einer Fragment-Bibliothek umfassend mindestens zwei, bevorzugt mindestens vier, sechs, acht, bevorzugt mindestens 10, 12, 20, 30, 40, 50, 60, 70, 80, 90 oder mindestens 100 Längenvarianten einer Nukleinsäure-Ausgangssequenz, gekennzeichnet dadurch dass
a) jede Längenvariante einen konstanten zentralen Bereich und jeweils einen 5'- und einen 3'- Bereich variabler Länge umfasst
b) jede Längenvariante mindestens aus einem ersten Teilfragment und einem zweiten Teilfragment zusammengesetzt wird, wobei jedes erste Teilfragment mindestens einen definierten 5'-Teilbereich des konstanten zentralen Bereiches und jedes zweite Teilfragment mindestens einen definierten 3'-Teilbereich des konstanten zentralen Bereiches umfasst.

Das Verfahren ist weiterhin dadurch gekennzeichnet, dass alle ersten und zweiten Teilfragmente in einem Schritt so miteinander verbunden werden, dass die Längenvarianten alle möglichen Kombinationen aus ersten und zweiten Teilfragmenten umfassen. In einer bevorzugten Ausführungsform werden die Teilfragmente so kombiniert, dass alle Längenvarianten zu annähernd gleichen Anteilen in der Fragment-Bibliothek vertreten sind. In einer besonders bevorzugten Ausführungsform können die Mengen der zu kombinierenden ersten und zweiten Teilfragmente direkt proportional zu ihrer Länge eingesetzt werden.

In besonderen Fällen kann eine weitere Kombination der ersten und zweiten Teilfragmente mit dritten oder vierten oder weiteren Teilfragmenten erwünscht sein. Dabei können die dritten oder vierten oder weiteren Teilfragmente z.B. aus einer völlig anderen Nukleinsäure-Ausgangssequenz oder einer zweiten gegenüber der ersten nur in bestimmten Teilbereichen modifizierten Nukleinsäure-Ausgangssequenz generiert werden. Beispielsweise können so Fragment-Bibliotheken aus verwandten Allelen oder Proteinvarianten oder aus verschiedenen Mitgliedern einer Proteinfamilie hergestellt werden.

Weiterhin ist das Verfahren dadurch gekennzeichnet, dass keine Kombination von ersten Teilfragmenten miteinander oder zweiten Teilfragmenten miteinander erfolgen kann, um eine gleichmäßige Verteilung der Fragmente in der Bibliothek zu gewährleisten.

In einer bevorzugten Ausführungsform werden die ersten und zweiten Teilfragmente der Fragment-Bibliothek über ein PCR-basiertes Verfahren mit spezifischen Oligonukleotiden hergestellt.

Beispielsweise kann die Herstellung der Teilfragmente durch Verlängerung von zu einem Teilbereich der Nukleinsäure-Ausgangssequenz homologen Oligonukleotiden durch ein PCR-basiertes Verfahren ("primer extension") erfolgen, wobei mindestens ungefähr 10, besser 12, bevorzugt mindestens 15 Nukleotide des 3'-Bereiches der Oligonukleotide zu dem definierten Teil der Nukleinsäure-Ausgangssequenz eine Homologie bzw. eine Identität von mindestens ungefähr 70%, 71%, 72%, 73%, 74%, bevorzugt mindestens 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% aufweisen und der 5'-Bereich der Oligonukleotide von der Nukleinsäure-Ausgangssequenz verschieden ist.

So kann die Gesamtlänge der zu einem Teilbereich der Nukleinsäure-Ausgangssequenz homologen Oligonukleotide ca. 30 Nukleotide, aber auch bis zu ungefähr 40 Nukleotide umfassen. Dabei kann beispielsweise der von der Nukleinsäure-Ausgangssequenz verschiedene 5'-Bereich eines Oligonukleotids für die Herstellung eines ersten Teilfragments folgendermaßen aufgebaut sein: einem 4 Nukleotid-Überhang folgt eine 6 Nukleotide umfassende Restriktionsschnittstelle sowie ein 3 Nukleotide umfassendes ATG-Startkodon. Somit ergäbe sich in diesem Fall für den 5'-Bereich eine Länge von 13 Nukleotiden. Der homologe 3'-Bereich sollte abhängig von den Eigenschaften des nicht homologen 5'-Bereiches des Oligonukleotids so lang sein, dass eine Bindung an den definierten Teil der Nukleinsäure-Ausgangssequenz möglich ist und eine PCR-basierte Verlängerung des Oligonukleotids erfolgen kann.

Zunächst wird der konstant zu haltende Zentralbereich innerhalb der Nukleinsäure-Ausgangssequenz festgelegt. Dieser kann bzgl. Länge und Region frei definiert werden. In einer bevorzugten Ausführungsform umfasst der Zentralbereich mindestens ungefähr 40 oder 45 Nukleotide, oder 50, 55, 60, 65, 70, 75, 80 oder 85 Nukleotide, besonders bevorzugt aber mindestens 90 oder 150 Nukleotide. Die Obergrenze des konstanten Zentralbereiches hängt von der Gesamtlänge der Nukleinsäure-Ausgangssequenz und der zugrunde liegenden Fragestellung ab. Dabei wird die Nukleinsäure-Ausgangssequenz zunächst in der Mitte des vorher definierten zentralen Bereiches in zwei Hälfen unterteilt, die als 5'- und 3'-Bereiche der Nukleinsäure-Ausgangssequenz bezeichnet sind.

Um die ersten und zweiten Teilfragmente herzustellen, wird für jedes erste und zweite Teilfragment ein separates Oligonukleotidpaar entworfen. Die Oligonukleotidpaare für die Herstellung der ersten Teilfragmente bestehen aus je einem trunkierenden forward (fwd) Oligonukleotid, welches mit seinem 3'-Ende an eine definierte Sequenz im 5'-Bereich der Nukleinsäure-Ausgangssequenz bindet und einem revers (rev) Gegen-Oligonukleotid, welches mit seinem 3'-Ende innerhalb des konstanten zentralen Bereiches der Nukleinsäure-Ausgangssequenz in entgegengesetzter Richtung bindet. Das trunkierende forward Oligonukleotid wird für die Herstellung der ersten Teilfragmente variiert, während immer das gleiche revers Gegen-Oligonukleotid verwendet wird. Ebenso bestehen die Oligonukleotidpaare für die Herstellung der zweiten Teilfragmente aus je einem trunkierenden revers Oligonukleotid, welches mit seinem 3'-Ende an eine definierte Sequenz im 3'-Bereich der Nukleinsäure-Ausgangssequenz bindet und einem forward Gegen-Oligonukleotid, welches mit seinem 3'-Ende innerhalb des konstanten zentralen Bereiches der Nukleinsäure-Ausgangssequenz in entgegen gesetzter Richtung bindet. Das trunkierende revers Oligonukleotid wird für die Herstellung der zweiten Teilfragmente variiert, während immer das gleiche forward Gegen-Oligonukleotid verwendet wird.

In einer bevorzugten Ausführungsform ist die Herstellung der ersten Teilfragmente durch folgende Schritte gekennzeichnet
a) Bereitstellen einer definierten Nukleinsäure-Ausgangssequenz
b) Bereitstellen von einem ersten Oligonukleotid für die Synthese eines ersten Teilfragments, wobei der 3'-Bereich des ersten Oligonukleotids zu einer definierten Nukleinsäuresequenz im 5'-Bereich der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist,
c) Bereitstellen von einem zweiten Oligonukleotid wobei der 3'-Bereich des zweiten Oligonukleotids zu einer definierten Nukleinsäuresequenz des konstanten zentralen Bereiches der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist
d) Inkubation der Nukleinsäure-Ausgangssequenz aus a) mit dem ersten Oligonukleotid aus b), dem zweiten Oligonukleotid aus c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
e) Bereitstellen von einem dritten Oligonukleotid für die Synthese eines weiteren ersten Teilfragments, wobei der 3'-Bereich des dritten Oligonukleotids zu einer definierten Nukleinsäuresequenz im 5'-Bereich der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist und sich in mindestens einem, bevorzugt in mindestens drei Nukleotiden von den 3'-Bereichen der ersten Oligonukleotide unterscheidet,
f) Inkubation der Nukleinsäure-Ausgangssequenz aus a) mit dem dritten Oligonukleotid aus e), dem zweiten Oligonukleotid aus c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
g) ggf. Bereitstellen von weiteren Oligonukleotiden für die Synthese von weiteren ersten Teilfragmenten wobei jeder 3'-Bereich jedes weiteren Oligonukleotids zu einer anderen definierten Nukleinsäuresequenz im 5'-Bereich der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist und sich in mindestens einem, bevorzugt in mindestens drei Nukleotiden von den 3'-Bereichen der ersten und dritten Oligonukleotide unterscheidet
h) Inkubation der Nukleinsäure-Ausgangssequenz aus a) mit jeweils einem weiteren Oligonukleotid aus g), dem zweiten Oligonukleotid aus c) und einem Polymerasesystem unter Bedingungen, die geeignet sind eine Hybridisierung der homologen 3'-Bereiche der Oligonukleotide an die Nukleinsäure-Ausgangssequenz und deren Verlängerung durch Polymerasekettenreaktion zu ermöglichen Wiederholung der Schritte g) und h), bis alle gewünschten ersten Teilfragmente synthetisiert sind

Weiterhin ist in einer bevorzugten Ausführungsform die Herstellung der zweiten Teilfragmente durch folgende Schritte gekennzeichnet
a) Bereitstellen einer definierten Nukleinsäure-Ausgangssequenz
b) Bereitstellen von einem ersten Oligonukleotid für die Synthese eines ersten Teilfragments, wobei der 3'-Bereich des ersten Oligonukleotids zu einer definierten Sequenz im 3'-Bereich der Nukleinsäure-Ausgangssequenz zu mindestens ungefähr 70%, bevorzugt mindestens 75% identisch ist,
c) Bereitstellen von einem zweiten Oligonukleotid wobei der 3'-Bereich des zweiten Oligonukleotids zu einer definierten Sequenz des konstanten zentralen Bereiches der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist
d) Inkubation der Nukleinsäure-Ausgangssequenz aus a) mit dem ersten Oligonukleotid aus b), dem zweiten Oligonukleotid aus c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
e) Bereitstellen von einem dritten Oligonukleotid für die Synthese eines weiteren zweiten Teilfragments, wobei der 3'-Bereich des dritten Oligonukleotids zu einer definierten Sequenz im 3'-Bereich der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist und sich in mindestens einem, bevorzugt drei Nukleotiden von den 3'-Bereichen der ersten Oligonukleotide unterscheidet,
f) Inkubation der Nukleinsäure-Ausgangssequenz aus a) mit dem dritten Oligonukleotid aus e), dem zweiten Oligonukleotid aus c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
g) ggf. Bereitstellen von weiteren Oligonukleotiden für die Synthese von weiteren zweiten Teilfragmenten wobei jeder 3'-Bereich jedes weiteren Oligonukleotids zu einer anderen definierten Sequenz im 3'-Bereich der Nukleinsäure-Ausgangssequenz zu mindestens ungefähr 70%, 71%, 72%, 73%, 74%, bevorzugt mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% identisch ist und sich in mindestens einem, bevorzugt mindestens drei Nukleotiden von den 3'-Bereichen der ersten und dritten Oligonukleotide unterscheidet
h) Inkubation der Nukleinsäure-Ausgangssequenz aus a) mit jeweils einem weiteren Oligonukleotid aus g), dem zweiten Oligonukleotid aus c) und einem Polymerasesystem unter Bedingungen, die geeignet sind eine Hybridisierung der homologen 3'-Bereiche der Oligonukleotide an die Nukleinsäure-Ausgangssequenz und deren Verlängerung durch Polymerasekettenreaktion zu ermöglichen Wiederholung der Schritte g) und h), bis alle gewünschten zweiten Teilfragmente synthetisiert sind.
   In einer besonders bevorzugten Ausführungsform des Verfahrens zur Herstellung einer Fragment-Bibliothek werden sowohl alle ersten als auch alle zweiten Teilfragmente gemäß dem beschriebenen Verfahren in separaten PCR-Reaktionen hergestellt. Die ersten und zweiten Teilfragmente können dabei nacheinander oder gleichzeitig z.B. in parallelen PCR-Reaktionen hergestellt werden.

In einer bevorzugten Ausführungsform erhalten die forward und revers Gegen-Oligonukleotide jeweils im 5'-Bereich eine Erkennungssequenz für ein Typlls-Restriktionsenzym, die so platziert ist, dass nach Restriktion der PCR-Produkte bzw. der Teilfragmente mit dem Typlls-Restriktionsenzym ein gemeinsamer nicht-palindromischer Überhang von optimalerweise mindestens vier Nukleotiden entsteht, der in den ersten und zweiten Teilfragmenten komplementär ist und einem Teil des zentralen Bereiches entspricht. Über die nicht-palindromischen Überhänge können die in separaten PCR-Reaktionen generierten ersten und zweiten Teilfragmente zusammenkombiniert werden.

Beschrieben wird somit ein Verfahren zum Herstellen einer Fragment-Bibliothek wobei die Herstellung aller ersten und zweiten Teilfragmente durch ein PCR-basiertes Verfahren unter Verwendung spezifischer Oligonukleotide erfolgt, wobei mindestens die forward und revers Gegen-Oligonukleotide welche im konstanten zentralen Bereich einer Ausgangs-Nukleinsäuresequenz binden, jeweils Erkennungssequenzen für dasselbe oder zwei verschiedene Typlls-Restriktionsenzyme enthalten, so dass der Restriktionsverdau aller ersten und zweiten Teilfragmente oder einer Auswahl davon nach Restriktion mit dem/den entsprechenden Enzym/en zur Generierung einzelsträngiger palindromischer oder bevorzugt nicht-palindromischer Überhänge an den 3' Enden der ersten und den 5' Enden der zweiten Teilfragmente führt und deren anschließendes Verbinden in gerichteter Orientierung durch Ligation oder Rekombination, so dass nur ein Verbinden von ersten mit zweiten Teilfragmenten möglich ist.
Für die Verkürzung des 5'-Bereiches der Nukleinsäure-Ausgangssequenz werden mehrere forward Oligonukleotide entworfen, die mit ihrem 3'-Ende jeweils so an eine definierte Sequenz des 5'-Bereiches der Nukleinsäure-Ausgangsequenz binden, dass die gewünschten ersten Teilfragmente mit Verkürzungen des 5'-Bereiches entstehen. Dabei kann jedes forward Oligonukleotid an seinem nicht hybridisierenden 5'-Ende Sequenzen, wie z.B. ein Start-Kodon oder zusätzliche regulatorische Sequenzen anhängen (falls diese nicht bereits im Zielvektor vorhanden sind) sowie eine Schnittstelle für die anschließende Klonierung in den Zielvektor.

Entsprechend werden für die Verkürzung des 3'-Bereiches der Nukleinsäure-Ausgangssequenz mehrere revers Oligonukleotide entworfen, die mit ihrem 3'-Ende jeweils so an eine definierte Sequenz des 3'-Bereiches der Nukleinsäure-Ausgangsequenz binden, dass die gewünschten zweiten Teilfragmente mit Verkürzungen des 3'-Bereiches entstehen. Dabei kann jedes revers Oligonukleotid an seinem nicht hybridisierenden 5'-Ende Sequenzen, wie z.B. ein Stopp-Kodon oder zusätzliche regulatorische Sequenzen anhängen (falls diese nicht bereits im Zielvektor vorhanden sind), sowie eine Schnittstelle für die anschließende Klonierung in den Zielvektor.

Die forward und revers Oligonukleotide weisen dabei in Abhängigkeit von der Länge und/oder Schmelztemperatur jeweils ein 3'-Ende auf, das mindestens um ungefähr 12, bevorzugt mindestens um 13, besonders bevorzugt mindestens um 18 Nukleotide mit dem jeweils festgelegten Bindebereich der Nukleinsäure-Ausgangsequenz überlappt, um eine ausreichende Hybridisierung der Oligonukleotide an die 5'- und 3'-Bereiche für die PCR-Amplifikation zu gewährleisten.

In einer bevorzugten Ausführungsform sind die Oligonukleotidpaare derart beschaffen, dass eine schrittweise Verkürzung der 5'- und 3'-Bereiche durch PCR-basierte Verlängerung ("prirner extension") der an die Nukleinsäure-Ausgangsequenz hybridisierten Oligonukleotide in separaten PCR-Reaktionen erfolgen kann. Die mittels der Oligonukleotide eingeführten Verkürzungen erfolgen dabei bevorzugt jeweils aminosäureweise bzw. auf Nukleotidebene in 3er-Schritten (kodonweise), um Leserastersprünge in den resultierenden Fragment-Bibliotheken zu vermeiden. In Abhängigkeit von der Gesamtlänge der Nukleinsäure-Ausgangssequenz kann die Verkürzung auch zunächst in 6er, 9er, 12er oder noch größeren Schritten erfolgen. Der Fachmann wird hier vorher abwägen, welche Verkürzungsstrategie je nach Applikation sinnvoll erscheint und ob ganze Abschnitte oder Domänen in Abhängigkeit von der jeweiligen Fragestellung übersprungen werden können.

Um eine Fragment-Bibliothek mit definierten Längenvarianten der Nukleinsäure-Ausgangssequenz zu erhalten, besteht die Möglichkeit, alle ersten mit allen zweiten Teilfragmenten zu kombinieren oder aber nur eine Mischung mit einer Auswahl oder Teilmenge an ersten oder zweiten Teilfragmenten bereitzustellen. Auch können verschiedene Mischungen erster und zweiter Teilfragmente hergestellt werden.

Um eine gleichmäßige Verteilung der Längenvarianten zu erzielen, kann vor Kombination der Teilfragmente deren Konzentration exakt vermessen werden (z.B. photometrisch bei einer Wellenlänge von 260 nm). Dann werden beispielsweise alle oder eine Teilauswahl der ersten und zweiten Teilfragmente in den gewünschten molaren Mengen (i.d.R. equimolar) vereinigt. Falls notwendig, können jedoch auch unterschiedliche Mengen der separat hergestellten Teilfragmente vereinigt werden, um den Anteil der entstehenden Längenvarianten zu beeinflussen. So kann es in besonderen Fällen von Vorteil sein, größere Mengen von längeren Teilfragmenten und geringere Mengen von kürzeren Teilfragmenten einzusetzen, um eine bevorzugte Entstehung von kürzeren Längenvariationen zu vermeiden.

In einer bevorzugten Ausführungsform des Verfahrens zur Herstellung der Fragment-Bibliotheken weisen die 3'-Enden der ersten Teilfragmente und die 5'-Enden der zweiten Teilfragmente einzelsträngige Überhänge auf und die ersten und zweiten Teilfragmente werden durch Ligation miteinander verbunden. Die Kombination der Teilfragmente kann dabei in An- oder Abwesenheit von Ligase oder Topoisomerase erfolgen oder alternativ auch durch in vitro Rekombination oder durch in vivo Rekombination in Abwesenheit von Ligase erfolgen. Dabei können die einzelsträngigen Überhänge der ersten und zweiten Teilfragmente entweder palindromisch oder in einer bevorzugten Ausführungsform nicht palindromisch sein.

In der besonders bevorzugten Ausführungsform werden die ersten und zweiten Teilfragmente aus der erhaltenen Mischung mit einem geeigneten Enzym zur Generierung einzelsträngiger Überhänge an den 3'-Enden der ersten und den 5'-Enden der zweiten Teilfragmente inkubiert, aufgereinigt und anschließend durch Ligation in Anwesenheit einer Ligase (z.B. T4-Ligase) verbunden. Bei dem Enzym kann es sich um eine Endonuklease wie eine Typll Restriktionsendonuklease oder in einer bevorzugten Ausführungsform eine Typlls Restriktionsendonuklease handeln.

Bei den Typlls Restriktionsenzymen kann es sich beispielsweise um Bbsl, Bsal, BsmBl, Earl, oder Ecil handeln. Andere passende Typlls Restriktionsenzyme können z.B. unter dem folgenden Link des Anbieters New England Biolabs® gefunden werden: http://www.neb.com/nebecomm/EnzymeFinder.asp?. Besonders bevorzugt handelt es sich um Restriktionsenzyme, die außerhalb ihrer Erkennungssequenz schneiden. Dabei können entweder alle ersten und zweiten Teilfragmente getrennt bzw. die Mischung aus ersten und zweiten Teilfragmenten getrennt mit verschiedenen Enzymen oder alle ersten und zweiten Teilfragmente in einem gemeinsamen Schritt mit demselben Enzym verdaut werden ("one pot reaction"), um die einzelsträngigen Überhänge zu erzeugen.

Da der durch das Typlls Enzym generierte Überhang in einer bevorzugten Ausführungsform nicht palindromisch ist, kann immer nur ein erstes Teilfragment mit einem zweiten Teilfragment ligieren. Durch die Ligation können alle möglichen Kombinationen aus ersten und zweiten Teilfragmenten generiert werden, wobei sich die Diversität aus dem Produkt der Anzahl der ersten und zweiten Teilfragmente berechnet. Alternativ werden die 5'- und 3'-Enden der ligierten Längenvarianten anschließend mit weiteren Restriktionsenzymen (z.B. Typll oder Typlls Enzyme) geschnitten, um einzelsträngige Überhänge für die gerichtete Klonierung der Längenvarianten in einen Zielvektor zu generieren. Dieser Verdau kann jedoch auch bereits parallel mit dem TypIIs-Verdau der Teilfragmente stattfinden.

Zur Erzeugung der einzelsträngigen Überhänge der ersten und zweiten Teilfragmente kann weiterhin ein Enzym mit einer Exonukleaseaktivität, wie z.B. Exonuklease III, Exonuklease T5, die lambda-Exonuklease oder wie z.B. die Exonukleaseaktivitäten von bestimmten DNA-Polymerasen eingesetzt werden. Bei der DNA-Polymerase kann es sich z.B. um eine T4-DNA-Polymerase, eine T7-DNA-Polymerase, DNA Polymerase I, Klenow-DNA-Polymerase, Pfu-Polymerase, Phi29-DNA-Polymerase, Phusion^{™}-High-Fidelity-Polymerase, VentR®, Deep VentR®, 9°-Nₘ-DNA-Polymerase oder eine Taq-Polymerase mit "proof reading"-Aktivität handeln. Entsprechende Verfahren sind z.B. in Aslanidis und de Jong, 1990, Nucl. Acids Res. 18:6069, WO07032837, WO09103027, oder WO07021944 beschrieben. Da im Falle eines Exonuklease-Verdaus auch Nukleotide in 3'-5'-Richtung von den nicht zu fusionierenden bzw. unterschiedlich verkürzten Enden der Teilfragmente entfernt werden, können die resultierenden 5'-Überhänge nach Kombination der ersten und zweiten Teilfragmente z.B. mit dem Klenow-Fragment der DNA Polymerase I wieder aufgefüllt werden. Alternativ können die resultierenden 5'-Überhänge zur Klonierung der Längenvarianten in einen Zielvektor dienen. Diese Möglichkeit kann beim Design der terminalen Oligonukleotide für die Herstellung der Teilfragmente mit berücksichtigt werden.

Alternativ können die einzelsträngigen Überhänge der ersten und zweiten Teilfragmente mit Hilfe des USER^{™} Enzyms (Mischung aus Uracil-DNA Glycosylase (UDG) und der DNA-Glycosylase-Lyase Endo VIII) von New England Biolabs (siehe US7435572) erzeugt werden. Dafür wird jeweils in die zweiten Oligonukleotide, welche bei der Herstellung der ersten und zweiten Teilfragmente durch PCR an den konstanten zentralen Bereich der Nukleinsäure-Ausgangssequenz hybridisieren ein Uracil-Rest eingebaut. Die PCR-Produkte bzw. die ersten und zweiten Teilfragmente werden anschließend mit dem USER-Enzym behandelt, welches den Uracil-Rest sowie die Nukleotide in 5'-Richtung entfernt, wodurch einzelsträngige 3'-Überhänge entstehen. Die kompatiblen einzelsträngigen Enden der ersten und zweiten Teilfragmente werden anschließend assembliert und falls nötig (bei kurzen kompatiblen Enden) in Anwesenheit einer Ligase ligiert.

In einer weiteren Ausführungsform des Verfahrens zur Kombination der ersten und zweiten Teilfragmente überlappen die 3'-Enden der ersten Teilfragmente mit den 5'-Enden der zweiten Teilfragmente mindestens um ungefähr 15, 16, 17, 18, 19 oder bevorzugt mindestens um 20 Nukleotide oder die Nukleinsäuresequenz der 3' Enden der ersten Teilfragmente ist mit der Nukleinsäuresequenz der 5' Enden der zweiten Teilfragmente zu mindestens ungefähr 70%, 71%, 72%, 73%, 74%, bevorzugt zu mindestens 75% oder 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, oder 100% homolog bzw. identisch, und die ersten und zweiten Teilfragmente werden über Fusions-PCR ("overlap extension" PCR) miteinander kombiniert. Dem Fachmann ist bekannt, dass ggf. auch noch eine Überlappung von weniger als 20 Nukleotiden bzw. eine Identität von etwas weniger als 75%, also z.B. 72 oder 73%, ausreichen kann, um eine Fusion der Teilfragmente zu erlauben. Dies hängt natürlich von der Beschaffenheit bzw. der Nukleotid-Zusammensetzung der 5'-Enden der zweiten Teilfragmente und der 3'-Enden der ersten Teilfragmente ab. Die Verwendung von Fusions-PCR zur Kombination von Teilfragmenten ist in folgenden Dokumenten näher beschrieben: Horton et al., 1989, Gene 15:61-8; Horton et al., 1990, Biotechniques 8:528-535, Pogulis et al., 2000, The Nucleic Acid Protocols Handbook Humana Press Inc., Totowa, NJ 2000 10.1385/1-59259-038-1:857.

Die Wahl des geeigneten Verfahrens zur Kombination der ersten und zweiten Teilfragmente hängt von verschiedenen Zielsetzungen und Bedingungen wie beispielsweise der Länge der Nukleinsäure-Ausgangssequenz und der davon abgeleiteten Längenvarianten, der Länge des konstanten zentralen Bereiches ab. So muss bei Kombination über Fusions-PCR oder Rekombination ein längerer Überlappungsbereich zwischen ersten und zweiten Teilfragmenten berücksichtigt werden als bei Kombination über Restriktionsverdau und Ligation.

Schließlich können die Teilfragmente bzw. die resultierenden Längenvarianten in geeignete Expressionsvektoren kloniert werden. In einer bevorzugten Ausführungsform geschieht dies über Restriktionsverdau der an den Enden der Teilfragmente angehängten Restriktionsschnittstellen - wie unter anderem im Beispiel beschrieben und anschließender Ligation in den über TypII oder TypIIs Verdau linearisierten Vektor. Die Klonierung der Teilfragmente in den Vektor kann in einem oder mehreren Schritten erfolgen. So kann z.B. zunächst die Kombination der Teilfragmente zu Längenvarianten stattfinden, bevor die Längenvarianten in den Zielvektor inseriert werden. Alternativ kann die Kombination der Teilfragmente mit dem Vektor gleichzeitig stattfinden. Ein geeignetes Verfahren für die gleichzeitige und gerichtete Assemblierung von mindestens zwei Fragmenten in einen Zielvektor in einer "ein-Schritt, ein-Topf"-Reaktion wird z.B. durch die sogenannte "Golden Gate"-Klonierung (Engler C, Kandzia R, Marillonnet S (2008) A One Pot, One Step, Precision Cloning Method with High Throughput Capability. PLoS ONE 3(11): e3647) ermöglicht. Dabei werden sowohl die zu inserierenden Fragmente als auch der Vektor mit Typlls Restriktionsenzymen verdaut, wodurch an allen Enden spezifische kompatible Überhänge erzeugt werden, die anschließend nur in einer bestimmten Kombination zusammengefügt werden können.

In einer weiteren Ausführungsform können die Längenvarianten durch Fusions-PCR in einen Zielvektor eingebracht werden, wenn die Enden der Längenvarianten mit den Enden eines linearisierten Vektors einen ausreichenden Überlappungsbereich aufweisen. Eine Insertion von Fragmenten in Vektoren über Fusions-PCR ist beispielsweise in dem sogenannten CPEC-Verfahren ("Circular Polymerase Extension Cloning", Quan J, Tian J (2009) Circular Polymerase Extension Cloning of Complex Gene Libraries and Pathways. PLoS ONE 4(7): e6441) beschrieben. Dieses Verfahren erlaubt sowohl die Insertion von einzelnen Längenvarianten in einen Zielvektor als auch die gleichzeitige Insertion von mehreren Teilfragmenten durch überlappende bzw. weitgehend homologe Bereiche zwischen (i) den äußeren Enden der Teilfragmente und den Enden des linearisierten Vektors sowie (ii) den einander zugewandten Enden der ersten und zweiten Teilfragmente im Bereich des konstanten zentralen Bereichs.

In einer weiteren Ausführungsform können die Längenvarianten oder Teilfragmente und der Vektor auch nach Erzeugen von einzelsträngigen überlappenden Enden an allen Molekülen mit Hilfe von Exonukleasen durch in vitro Rekombination assembliert werden. Eine geeignete Methode ist beispielsweise das sogenannte "SLIC"-Verfahren, welches eine Sequenz- und Ligations-unabhängige Klonierungsmethode für eine ein-Schritt Assemblierung mehrerer Moleküle beschreibt, bei dem z.B. die T4 Exonuklease zur Erzeugung einzelsträngiger Überhänge eingesetzt wird. (Li MZ, Elledge SJ. Harnessing homologous recombination in vitro to generate recombinant DNA via SLIC. Nat Methods 2007 Mar;4(3):251-6). Weitere geeignete Exonukleasen für entsprechende Verfahren sind an anderer Stelle in diesem Dokument erwähnt. Die Hybridisierung der einzelsträngigen Enden kann z.B. durch RecA unterstützt werden.

Schließlich können in einer weiteren Ausführungsform die Längenvarianten oder Teilfragmente auch mit Hilfe der Gateway® Cloning Technologie durch in vitro Rekombination über att-sites in entsprechende Vektoren kloniert werden. Geeignete Vektoren und Verfahren sind dem Fachmann bekannt und beispielsweise in US5888732, US6143557, US6171861, US6270969 oder US6277608 beschrieben.

Auch können die oben genannten Verfahren miteinander kombiniert werden, z.B. Kombination der Teilfragmente über Typlls Verdau und Ligation und anschließende Rekombination der Längenvarianten in Gateway-Vektoren oder z.B. Kombination der Teilfragmente über über Typlls Verdau und Ligation und anschließende Insertion der Längenvarianten in den Zielvektor durch Fusions-PCR etc.

Gängige Vektoren sind z.B. pET, pBAD, pQE, pTrcHis, pGEX etc. Die die Längenvarianten enthaltenden Vektoren können dann separat oder als definierte Mischung von Vektoren bzw. als Fragment-Bibliothek in ein geeignetes prokaryontisches (z.B. Escherichia coli, Bacillus, Caulobacter, Lactobacillus, Lactococcus, Salmonella, Streptomyces) oder ein eukaryontisches Expressionssystem (z.B. Fadenpilze, Baculovirus/Pflanzen-, Hefe-, Insekten-, oder Säugerzellen) eingebracht werden, welches die verkürzten Polypeptid-Varianten bzw. die Polypeptid-Bibliothek produziert. Gängige Expressionssysteme und ihre jeweiligen Vor- und Nachteile sind z.B. in Schirrman et al. 2008, Frontiers in Bioscience 4576-4594 oder in Brondyk et al. 2009, Methods in Enzymology, 463:131-147 beschrieben. Über ein entsprechendes Selektionsverfahren werden anschließend geeignete Kandidaten mit gewünschten Eigenschaften aus der Polypeptid-Bibliothek selektiert.

Somit umfasst das Verfahren auch die Herstellung einer Polypeptid-Bibliothek gekennzeichnet durch die folgenden Schritte
a) Einbringen der Fragment-Bibliothek in geeignete Expressionsvektoren
b) Einbringen der Expressionsvektoren in eukaryontische oder prokaryontische Zellen
c) Kultivieren der Zellen unter Bedingungen, die geeignet sind, eine Expression der Fragment-Bibliothek zu erlauben
d) und ggf. Isolieren der Polypeptid-Bibliothek
e) und ggf. Screenen der Polypeptid-Bibliothek in einem geeigneten Selektionsverfahren zur Identifikation eines oder mehrerer Kandidaten mit gewünschten Eigenschaften

Alternativ kann die Fragment-Bibliothek in einem zellfreien System exprimiert und anschließend aufgereinigt oder isoliert werden. Bevorzugt handelt es sich dabei um ein in vitro Transkriptions-Translationssystem. Geeignete zellfreie Systeme zur Proteinproduktion z.B. aus Insektenzell-Lysat (Ezure et al., 2010, Curr Pharm Biotechnol. 11:279-84) aus "wheat germ" (Takai et al., 2010 Curr Pharm Biotechnol. 11:272-8), aus Kaninchen-Reticulozyten (Promega) oder aus E.coli (RTS-Lysat) sowie synthetische Systeme wie beispielsweise das PURESYSTEM® von BioComber (synthetisches Lysat mit E.coli Ribosomen), welches von Takuya Ueda entwickelt wurde (Shimizu und Ueda, 2010, Methods Mol Biol. 607:11-21) oder darauf basierende Systeme (z.B. WO2005105994) sind dem Fachmann bekannt.

Somit umfasst das Verfahren auch die Herstellung einer Polypeptid-Bibliothek gekennzeichnet durch die folgenden Schritte
a) Einbringen der Fragment-Bibliothek in geeignete Expressionsvektoren
b) Inkubation der Expressionsvektoren mit einem geeigneten in vitro Transkriptions-Translationssystem
c) ggf. Isolieren der Polypeptid-Bibliothek
d) und ggf. Screenen der Polypeptid-Bibliothek in einem geeigneten Selektionsverfahren zur Identifikation eines oder mehrerer Kandidaten mit gewünschten Eigenschaften

Mit Hilfe der Polypeptid-Bibliothek können funktionelle Domänen oder Aminosäure-Bereiche identifiziert, Epitope gemappt, Inhibitoren gescreent oder funktionelle verkürzte Signalpeptide generiert und selektiert werden. Außerdem kann die Polypeptid-Bibliothek zum Screenen von Enzymen mit verbesserten Eigenschaften verwendet werden, welche sich beispielsweise - wie oben ausgeführt - für Kristallisierungsstudien eignen. Darüber hinaus können neue funktionelle Minimalproteine generiert und selektiert werden.

Desweiteren können die Fragment-Bibliotheken auch nur transkribiert werden, falls beispielsweise RNA-Sequenzen untersucht oder gescreent werden sollen. Zu diesem Zweck kann ein kommerzielles zellfreies in vitro Transkriptionssystem wie z.B. das RiboMAX™ "Large Scale RNA Production System-T7" (US5256555, US6586218 und US6586219) verwendet werden.

Somit umfasst das Verfahren auch die Herstellung einer RNA-Bibliothek gekennzeichnet durch die folgenden Schritte
a) Einbringen der Fragment-Bibliothek in geeignete Expressionsvektoren
b) Inkubation der Expressionsvektoren mit einem geeigneten in vitro Transkriptionssystem
c) ggf. Isolieren der RNA-Bibliothek
d) und ggf. Screenen der RNA-Bibliothek in einem geeigneten Selektionsverfahren zur Identifikation eines oder mehrerer Kandidaten mit gewünschten Eigenschaften

Typische Zell- oder Virus-basierte Selektionsverfahren für die nach dem beschriebenen Verfahren hergestellten Polypeptid-Bibliotheken sind z.B. Bakterielles Display (Kemp, 1981, Proc. Natl. Acad. Sci. USA 78(7): 4520-4524) oder bakterielles Display über Bacillus Endosporen (s. Kim und Schuhmann, 2009, Cell Mol. Life Sci. 66:3127-3136), Yeast Two Hybrid (z.B. beschrieben in US5283173), Phagen Display (z.B. Sidhu et al., 2003, Chembiochem 4:14-25; US5223409, US5403484, US5571698, und US5837500), Yeast Surface Display (beschrieben z.B. in, WO08118476, WO09093118, Chien et al., 1991, Proc Natl Acad Sci USA 88:9578-82; Boder und Wittrup, 1997, Nat. Biotechnol. 15:553-557), oder retrovirale oder lentivirale Display-Verfahren (z.B. Buchholz et al., 2008, Comb Chem 11:99-110; Taube et al., 2008, PLoS 3:e3181). Alternativ können durch in vitro Transkription/Translation auch zellfreie Selektionssysteme wie z.B. mRNA Display (siehe z.B. WO98/54312 PROFUSION^{™}, US6258558, US6518018, US6281344, US6214553, US6261804, US6207446, US6846655, US6312927, US6602685, US6416950, US6429300, US7078197, und US6436665), ribosomales Display (siehe z.B. WO0175097, US5643768, US5658754, und US7074557) oder Mikrokompartimentierung (Sergeeva et al., 2006, Adv Drug Deliv Rev. 58(15):1622-1654) zur Identifizierung von verbesserten Polypeptid-/oder Proteinkandidaten zur verwendet werden. Viele dem Fachmann bekannten Display-Systeme und die jeweiligen Anwendungsgebiete sind beispielsweise in dem Übersichtsartikel Seergeva et al., 2006, Adv. Drug Deliv. Rev. 58:1622-1654 beschrieben.
Die Offenbarungen der vorangestellten Dokumente werden hiermit durch Inbezugnahme Bestandteil der vorliegenden Patentschrift.

Weiterhin kann die Polypeptid-Bibliothek zum Screenen von löslichen Polypeptidvarianten, eingesetzt werden. Im Stand der Technik sind verschiedene Verfahren zur Identifizierung von löslichen Proteinderivaten aus entsprechenden Fragment-Bibliotheken beschrieben. Häufig werden die entsprechenden Fragmente oder Varianten mit einem Reportergen fusioniert, dessen Funktion in Abhängigkeit von der löslichen Expression und korrekten Faltung des fusionierten Zielproteins oder Fragments einfach nachgewiesen werden kann. Als Reportergene dienen beispielsweise das grün fluoreszierende Protein (GFP) (Waldo et al., 2003, Curr Opin Chem Biol. 7:33-8, Coleman et al., 2004, J. Proteome Res., 3:1024-1032), die Chloramphenicol-Acetyl-Transferase (CAT) (Maxwell et al., 1999, Protein Science 8:1908-1911), Beta-Galactosidase (β-Gal) oder das Biotin-Carboxyl Carrier Protein (BCCG) (WO03064656). Um die Nebenwirkungen großer Fusionsanteile zu vermeiden, werden alternativ oft kleinere Bereiche oder Fragmente von Reportergenen an die Zielsequenz fusioniert, welche nach Expression des Fusionskonstruktes erst durch selbst-Komplementierung mit dem für die Reporterfunktion notwendigen fehlenden Fragment aktiviert werden. Entsprechende Ansätze sind z.B. in Ullmann et al., 1967; Nixon und Benkovic, 2000, Protein Eng. 13(5):323-7; Wigley et al., 2001, Nat Biotechnol. 19:131-6; Wehrman et al., 2002, Proc. Natl. Acad. Sci. U. S. A. 99:3469-74 (Komplementierung des lacZ-Fragments mit lacZO) oder in WO05074436 (Komplementierung eines kleinen fusionierten GFP-Anteils mit dem großen GFP-Fragment) beschrieben. Ein alternatives Verfahren zur Selektion von löslichen Proteinen offenbart WO06024875. Dabei wird die Fragment-Bibliothek mit einen kleinen Peptidsubstrat fusioniert, welches nur in Verbindung mit einem löslichen Fusionspartner von einem Enzym spezifisch modifiziert werden kann.

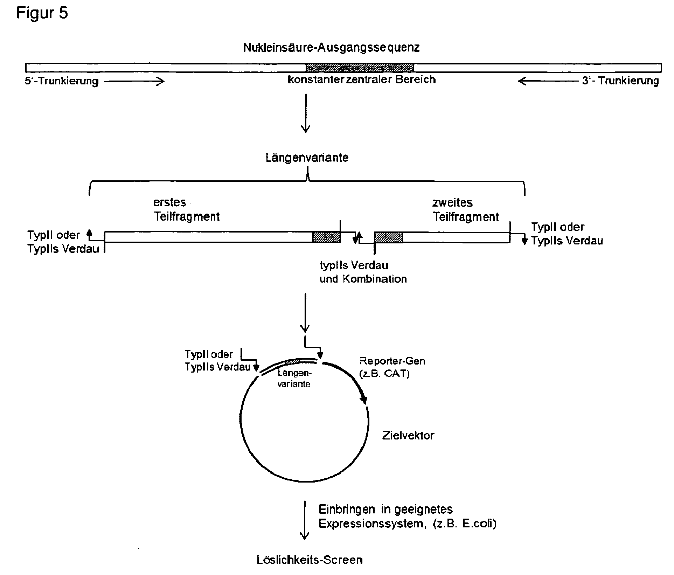

Offenbart ist auch die Verwendung der beschriebenen Fragment- bzw. Polypeptid-Bibliothek zum Screenen von löslichen Polypeptidvarianten oder Enzymen, wobei die Teilfragmente bzw. Längenvarianten in Fusion mit einem 3' gelegenen Reportergen in den Zielvektor kloniert werden, wobei das Reporter-Gen z.B. für eine Chloramphenicol-Acetyl-Transferase kodiert, und wobei das Reporter-Gen nur nachweislich exprimiert wird, falls die fusionierte Längenvariante löslich ist. Die Fusion von Teilfragmenten bzw. Längenvarianten mit dem Reporter-Gen kann bereits vor Klonierung in den Zielvektor erfolgen. Alternativ kann sich das Reporter-Gen bereits im Zielvektor befinden und die Fusion findet durch Klonieren der Teilfragmenten bzw. Längenvarianten in den Zielvektor statt. Eine mögliche Ausführungsform der Erfindung ist in Figur 5 dargestellt.

In einer besonderen Ausführungsform wird zwischen der Längenvariante und dem Reportergen ein Stopp Codon (z.B. amber TAG) eingebracht. Das erste Screening auf Löslichkeit erfolgt dann durch Transformation der in den Reportervektor ligierten Längenvarianten in einen amber Suppressor Stamm und Plattierung auf Selektionsplatten (z.B. in dieser Ausführungsform Chloramphenicol-Selektionsplatten). Das Stopp Codon im Fusionsbereich der beiden Fusionspartner wird in einem Suppressor-Stamm überlesen und es kommt zur Translation eines kompletten Fusionsproteins. Bevorzugt wird die Chloramphenicol Konzentration der Platten so gewählt, dass nur Kolonien wachsen können, die lösliche Fusionsprodukte aus Längenvariante + Chloramphenicol-Acetyl-Transferase exprimieren. Alternativ kann zusätzlich eine Unterscheidung in große und kleine Kolonien erfolgen, wobei zu erwarten ist, dass große Kolonien tendenziell mehr lösliches Fusionsprotein exprimieren. Um in einem zweiten Schritt die Löslichkeit der Längenvariante auch ohne Chloramphenicol-Acetyl-Transferase-Anteil zu verifizieren, kann isolierte Plasmid-DNA aus positiv selektierten Kolonien in einen nicht-Suppressor-Stamm retransformiert werden. Hier führt das amber Stopp Codon immer zu einer Terminierung der Expression und die Längenvariante wird ohne Fusionsanteil translatiert. Dabei ist zu beachten, dass der Zielselektionsvektor eine zweite, Chloramphenicol-unabhängige Resistenzkassette zur Etablierung transformierter Zellen enthält. Die Verifizierung der Löslichkeit der so exprimierten Längenvarianten kann daraufhin über gängige proteinbiochemische Verfahren erfolgen (z.B. Gelchromatografie, Affinitätschromatografie, Western Blot, ELISA).

Außerdem können die Fragment-Bibliotheken als solche, welche Längenvarianten nicht-kodierender oder regulatorischer Sequenzen aufweisen zum Screenen von funktionellen oder regulatorischen Elementen (z.B. Minimalpromotoren zur Verbesserung der Expression oder Minimalterminatoren) oder nicht regulatorischen Sequenzmotiven (z.B. untranslatierte Bereiche, Transposons oder Minimalintrons die gut gespleißt werden) verwendet werden.

### Figuren

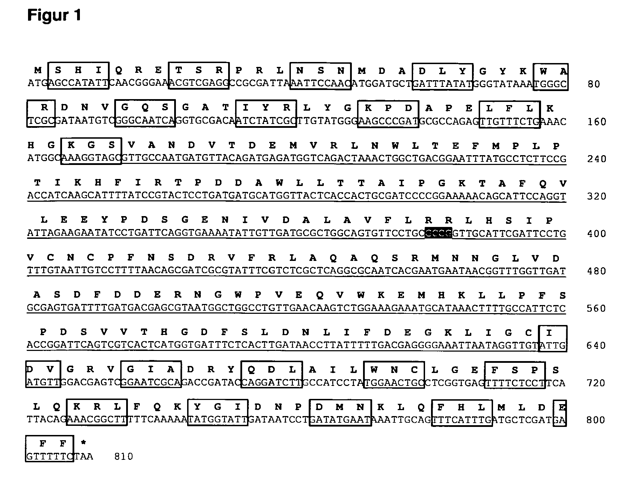

Die Erfindung wird weiter durch folgende Figuren erläutert
**Figur 1**: Gensequenz von nptll mit Angabe der kodierten Aminosäuresequenz im Einbuchstabencode. Zusätzlich über die Oligonukleotide eingeführte Start (ATG)- und Stopp (TAA)-Kodons sind angegeben, und der in jeder Längenvariante konstant gehaltene zentrale Bereich ist unterstrichen. Die Verkürzungen der Nukleinsäure-Ausgangssequenz in 3 Kodon bzw. 3-Aminosäureschritten ist durch umrahmte und nicht umrahmte Hinterlegung der 5'- und 3'-Bereiche angedeutet. Der 4 Nukleotide umfassende Überhang für die Kombination der ersten und zweiten Teilfragmente ist schwarz hinterlegt.

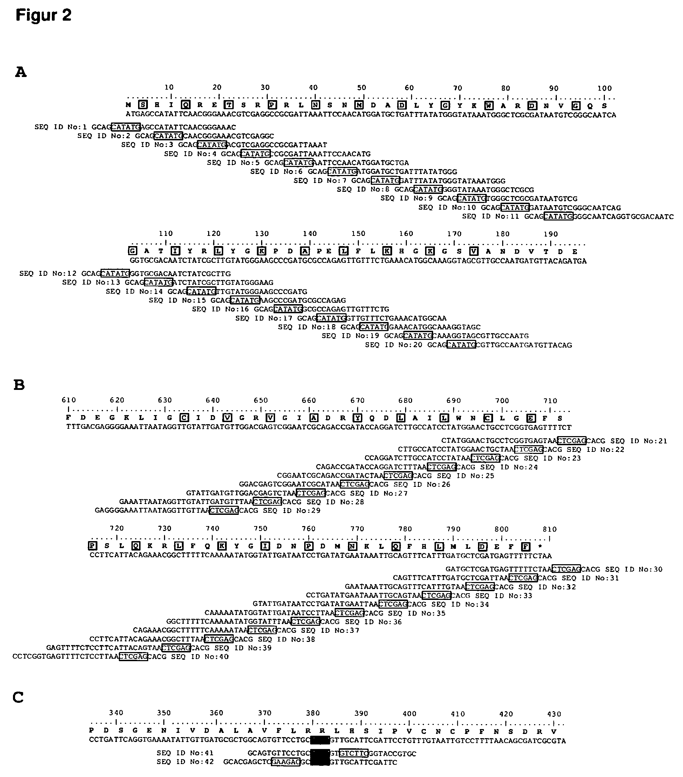

**Figur 2**: Teilabbildung A zeigt die Herstellung der ersten Teilfragmente des nptII-Gens durch schrittweise Verkürzung des 5'-Bereiches der Nukleinsäure-Ausgangssequenz mit Hilfe entsprechend konstruierter forward Oligonukleotide (SEQ ID NO:1-20), die alle ein Startkodon und eine Schnittstellenerkennungssequenz enthalten. Teilabbildung B zeigt die Herstellung der zweiten Teilfragmente des nptII-Gens durch schrittweise Verkürzung des 3'-Bereiches der Nukleinsäure-Ausgangssequenz mit Hilfe entsprechend konstruierter reverser Oligonukleotide (SEQ ID NO:21-40), die alle ein Stoppkodon und eine Schnittstellenerkennungssequenz enthalten. Teilabbildung C zeigt die für beide Teilfragmente verwendeten Gegenoligonukleotide (SEQ ID NO:41 und 42), die überlappend innerhalb des zentralen Bereiches der Nukleinsäure-Ausgangssequenz hybridisieren und jeweils die Typlls-Restriktionsschnittstelle Bbsl zur Generierung nicht palindrom ischer einzelsträngiger Überhänge enthalten.

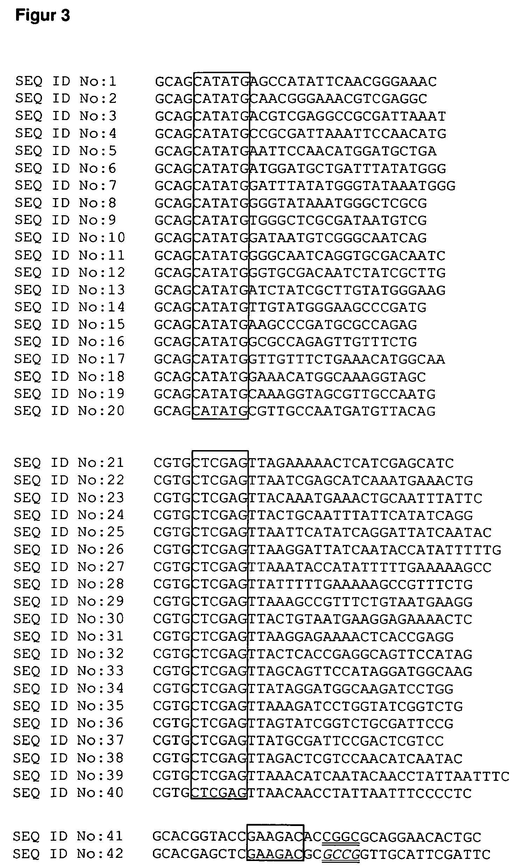

**Figur 3**: Oligonukleotide zur Herstellung der Teilfragmente für die nptll Fragment-Bibliothek. Die über die forward Oligonukleotide (SEQ ID NO:1-20) angefügte Ndel-Schnittstelle (CATATG) und die über die reversen Oligonukleotide (SEQ ID NO:21-40) angefügte Schnittstelle für Xhol (CTCGAG) sowie die Bbsl Erkennungssequenzen in den Gegen-Oligonukleotiden (revers: SEQ ID NO:41 und forward: SEQ ID NO:42) sind eingerahmt. Der durch Restriktion mit Bbsl generierte Überhang ist doppelt unterstrichen.

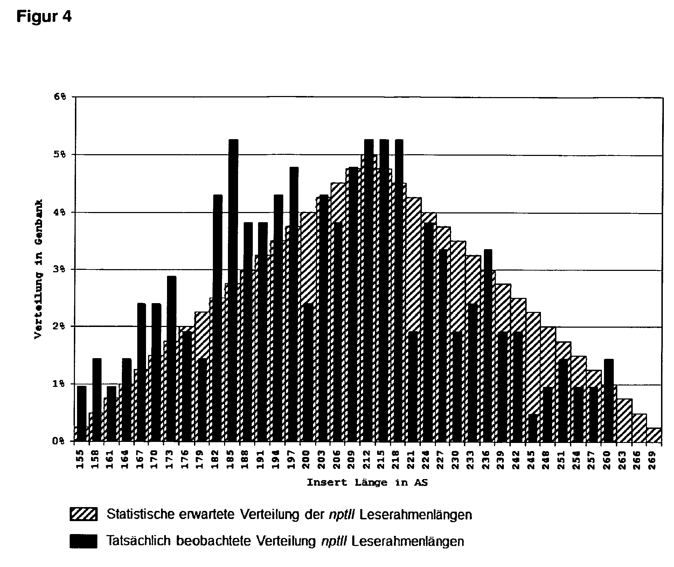

**Figur 4****:** Verteilung der nptII Längenvarianten innerhalb der Fragment-Bibliothek: Die statistisch erwartete Verteilung der der nptll-Leserahmenlängen ist weiß-schwarz schraffiert und die tatsächlich beobachtete Verteilung der nptll-Leserahmenlängen in schwarz dargestellt.
Figur 5: Herstellung einer Fragment-Bibliothek zur Identifizierung löslicher Polypeptidvarianten: basierend auf einer Nukleinsäure-Ausgangssequenz, welche für ein zu analysierendes Protein oder Polypeptid kodiert, werden nach Definition eines konstanten Bereichs (schraffiert, z.B. minimaler funktioneller Bereich) mit Hilfe spezifischer Oligonukleotide in separaten PCR-Verfahren erste und zweite Teilfragmente generiert, die die gewünschten 5'- und/oder 3`-Verkürzungen sowie die Hälfte des konstanten zentralen Bereichs enthalten. Jedes erste Teilfragment enthält am 3'-Ende und jedes zweite Teilfragment am 5'-Ende (jeweils im konstanten zentralen Bereich) eine spezifische Typlls Schnittstelle. Nach Restriktionsverdau werden die entstandenen einzelsträngigen Überhänge der ersten und zweiten Teilfragmente zu Längenvarianten verbunden (z.B. durch Ligation oder Rekombination). In der dargestellten Ausführungsform wurden die ersten Teilfragmente am 5'-Ende und die zweiten Teilfragmente am 3'-Ende jeweils mit weiteren Typll oder Typlls Schnittstellen versehen, um ein anschließendes Einklonieren der erhaltenen Längenvarianten in einen Zielvektor zu ermöglichen. Der Vektor enthält ein Reportergen (in diesem Fall ein CAT-Gen) welches nach Insertion der Längenvarianten in einem Reporter-Fusionsprotein resultiert. Optional kann die Fusionsstelle über ein amber Stopp Codon unterbrochen sein, um je nach verwendetem Stamm eine Expression des Fusionsproteins oder der Längenvariante allein zu ermöglichen. Die Vektoren, welche die verschiedenen Längenvarianten enthalten, können anschließend in geeignete Expressionssysteme, wie z.B. E.coli eingebracht werden, um die exprimierten verkürzten Polypeptidvarianten abhängig von der Expressionseffizienz auf Löslichkeit zu testen.

### Beispiel

In diesem Ausführungsbeispiel wurde das Gen nptII (SEQ ID NO:43) welches für eine Kanamycin Resistenz-vermittelnde Aminoglycosid-3'-Phosphotransferase des Typs II kodiert (SEQ ID NO:44) und als Nukleinsäure-Ausgangssequenz diente, in 2 x 20 Schritten von je 3 Kodons (bzw. 3 Am inosäuren) jeweils vom 5'- und 3'-Ende verkürzt. (Figur 1 alternierend grau/weiß hinterlegt). Da die Kombinationen der 20 ersten Teilfragmente mit den 20 zweiten Teilfragmenten zufällig sind, ergibt sich eine Gesamtzahl von 20 x 20 = 400 möglichen Kombinationen. Bei der Verkürzung um je 3 Kodons wurden das Start- und Stoppkodon (Figur 1, eingerahmt) nicht mitgezählt, sondern vielmehr an jedes Teilfragment mit angehängt, um eine korrekte Translation auch der verkürzten Längenvarianten zu gewährleisten. Demzufolge wurde für alle Längenvarianten ein konstanter zentraler Bereich von 154 Kodons (kodierend für VANDV...KLIGC, in Figur 1 unterstrichen) festgelegt. Für die spätere Kombination der ersten und zweiten Teilfragmente wurde ein nicht-palindromischer Sequenzbereich von 4 bp ungefähr in der Mitte des zentralen Bereiches gewählt (GCCG, in Figur 1 und 2 jeweils schwarz hinterlegt).

Für die Herstellung aller 20 ersten Teilfragmente, welche die Verkürzungen des 5'-Bereiches der Nukleinsäure-Ausgangssequenz repräsentieren und alle 20 zweiten Teilfragmente, welche die Verkürzungen des 3'-Bereiches der Nukleinsäure-Ausgangssequenz repräsentieren, wurden die Oligonukleotidpaare so gewählt, dass mit jedem Oligonukleotidpaar über PCR eines der Teilfragmente in der gewünschten Größe amplifiziert werden konnte. Dabei wurde bei der Herstellung der ersten Teilfragmente zusätzlich je ein Start-Kodon, sowie die Klonierungsschnittstelle Ndel über die Oligonukleotide angehängt (Figur 2A) und entsprechend bei der Herstellung der zweiten Teilfragmente zusätzlich je ein Stopp-Kodon, sowie die Klonierungsschnittstelle Xhol über die Oligonukleotide angehängt (Figur 2B). Innerhalb des zentralen Bereiches wurde für die Herstellung der ersten und zweiten Teilfragmente jeweils ein Oligonukleotid so gewählt, dass dieses als Gegen-Oligonukleotid für die 5'-forward-bzw. die 3'-revers Oligonukleotide fungierte. Diese beiden mit einem Teil des zentralen Bereiches hybridisierenden Oligonukleotide (SEQ ID NO:41 und 42) beinhalten den nicht-palindromen Sequenzbereich von 4 Basenpaaren für die Ligation, gefolgt von je einer Bbsl-Schnittstelle (Figur 2C). Das Restriktionsenzym Bbsl gehört zu den Typlls-Enzymen, die außerhalb ihrer Erkennungssequenz schneiden. So lautet beispielsweise die Erkennungssequenz für Bbsl GAAGACNN'XXXX. In diesem Fall stellt XXXX den nicht-palindromen Sequenzbereich für die Ligation dar (GCCG).

Die für die Herstellung der ersten 20 und zweiten 20 Teilfragmente benötigten Oligonukleotide sind in Figur 3 aufgelistet (SEQ ID NO:1-42). Mit diesen Oligonukleotiden wurden nach dem unten angegebenen Schema 40 PCR-Reaktionen nach einem Standardprotokoll angesetzt (PCR01 bis PCR20 für die Herstellung der ersten Teilfragmente und PCR21 bis PCR40 für die Herstellung der zweiten Teilfragmente). Als Nukleinsäure-Ausgangssequenz wurde jeweils das synthetisch hergestellte Gen nptll (SEQ ID NO:43) verwendet.
PCR01: SEQ ID NO:1 und SEQ ID NO:41
PCR02: SEQ ID NO:2 und SEQ ID NO:41
...
PCR20: SEQ ID NO:20 und SEQ ID NO:41

PCR21: SEQ ID NO:42 und SEQ ID NO:21
PCR22: SEQ ID NO:42 und SEQ ID NO:22
...
PCR40: SEQ ID NO:42 und SEQ ID NO:40

Die PCR-Produkte wurden unter Verwendung von Säulchen mit einer Silica-Membran aufgereinigt und anschließend deren Konzentration photometrisch bestimmt. Gleiche molare Mengen wurden vereinigt, mit dem Typlls Restriktionsenzym Bbsl geschnitten und erneut aufgereinigt. Die gesamte Mischung der verdauten ersten und zweiten Teilfragmente wurde mit Ligase versetzt und 3 h bei Raumtemperatur inkubiert, wobei jedes erste Teilfragment mit jedem zweiten Teilfragment kombinieren konnte. Da die von der Bbsl Endonuklease generierten Überhänge nicht palindrom isch sind, wurden die unerwünschten Kombinationen von ersten mit ersten Teilfragmenten und von zweiten mit zweiten Teilfragmenten vermieden. Die Reaktion wurde durch einen Hitzeschritt abgestoppt und die ligierten Längenvarianten erneut gereinigt, anschließend mit den TypII Restriktionsenzymen Ndel und Xhol geschnitten und in den ebenso geschnittenen Zielvektor pEG-His1 ligiert. Der Ligationsansatz wurde in kompetente E. coli DH10 B Zellen transformiert und sowohl auf Ampicillin- als auch auf Kanamycin/IPTG-Selektionsmedium ausplattiert. Bei dem Vektor pEG-His1 handelt es sich um einen durch IPTG induzierbaren Expressionsvektor, der zu Selektionszwecken eine konstitutive Ampicillin-Resistenz trägt. Demzufolge war zu erwarten, dass alle Zellen, die einen Vektor aus dem Ligationsansatz erhalten hatten, auf Ampicillin-Platten wachsen konnten. Dahingegen sollten jedoch nur solche Zellen Kanamycin-resistent sein, die einen rekombinanten Vektor trugen, dessen nptII Gen für eine funktionale Aminoglycosid-3'-Phosphotransferase kodierte, und in welchem die entsprechenden amino- und/oder carboxyl-terminalen Verkürzungen die Aktivität des Proteins nicht beeinträchtigten.
Demzufolge erlaubte die Stichprobenanalyse der auf Ampicillin wachsenden Kolonien eine Aussage über die neutrale Gesamtverteilung der partiell verkürzten nptII Gene innerhalb der gesamten Fragment-Bibliothek. Die Stichprobenanalyse der auf Kanamycin wachsenden Kolonien dagegen ermöglichte die funktionelle Analyse der Genbank, indem die Minimalgröße der aktiven Aminoglycosid-3'-Phosphotransferase bestimmt wurde.

Insgesamt wurde das nptll Gen aus 209 Ampicillin-resistenten Kolonien sequenzanalysiert. Die statistische Verteilung der Gesamtlänge des nptll Leserahmens entsprach der erwarteten Verteilung, mit der kleinen Einschränkung, dass kürzere Fragmente leicht überrepräsentiert waren (Figur 4). Dies liegt vermutlich an der höheren Ligationseffizienz kürzerer Fragmente und könnte durch entsprechende Steigerung der eingesetzten Menge der längeren ersten und zweiten Teilfragmente in der initialen Ligation kompensiert werden. Die separate Betrachtung der Verkürzungen der 5'- und 3'-Bereiche der Nukleinsäure-Ausgangssequenz zeigte, dass alle erwarteten Einzelverkürzungen in der unselektierten Stichprobe von 209 Stück gefunden wurden.

Die Sequenzanalyse der Kanamycin-resistenten Kolonien ergab für den Carboxyl-Terminus, dass die letzten drei Aminosäuren für die Funktionalität des Proteins essentiell sind. (Ein detaillierteres Ergebnis auf Einzelaminosäure-Ebene könnte in einem weiteren Versuch unter Verwendung einer entsprechenden Feinraster Fragment-Bibliothek mit Verkürzungen von jeweils nur einer Aminosäure gewonnen werden). Für den Amino-Terminus ist hingegen eine Verkürzung von bis zu 21 Aminosäuren für die Aktivität des Enzyms tolerierbar. Insgesamt definiert das Experiment als Minimalmotiv für die Aminoglycosid-3'-Phosphotransferase Nptll die Aminosäuresequenz [MGYKWAR...MLDEFF]. Diese Information kann genutzt werden, um funktionelle Proteinvarianten mit neuen Eigenschaften (etwa veränderte Löslichkeit) herzustellen, oder das Gen für eine funktionierende Kanamycin-Resistenz in Vektoren um 63 Nukleotide zu verkürzen. Das erfindungsgemäße Verfahren zur Herstellung von Fragment-Bibliotheken erlaubt somit eine erhöhte Screening Effizienz durch einen größeren Anteil korrekter bzw. funktioneller Klone, wodurch Ressourcen beim Screenen von optimalen Kandidaten eingespart werden können.

## Patentansprüche

1. Verfahren zur Herstellung einer Fragment-Bibliothek umfassend mindestens zwei, bevorzugt mindestens vier Längenvarianten einer Nukleinsäure-Ausgangssequenz, **gekennzeichnet dadurch dass**
a) jede Längenvariante einen konstanten zentralen Bereich und jeweils einen 5'- und einen 3'- Bereich variabler Länge umfasst,
b) jede Längenvariante mindestens aus einem ersten Teilfragment und einem zweiten Teilfragment zusammengesetzt wird, wobei jedes erste Teilfragment mindestens einen definierten 5'-Teilbereich des konstanten zentralen Bereiches und jedes zweite Teilfragment mindestens einen definierten 3'-Teilbereich des konstanten zentralen Bereiches umfasst und die ersten und zweiten Teilfragmente keine Leserastermutationen gegenüber der Nukleinsäure-Ausgangssequenz aufweisen,
c) wobei alle ersten und zweiten Teilfragmente in einem Schritt so miteinander verbunden werden, dass die Längenvarianten alle möglichen Kombinationen aus ersten und zweiten Teilfragmenten aufweisen,
d) und optional keine Kombination von ersten Teilfragmenten miteinander oder zweiten Teilfragmenten miteinander erfolgen kann.

2. Verfahren nach Anspruch 1, wobei der 5'-Bereich jeder Längenvariante durch X Nukleotide definiert ist und der 3'- Bereich jeder Längenvariante durch Y Nukleotide definiert ist, X und Y ganze Zahlen sind und der zentrale Bereich in jeder Längenvariante identisch ist, **dadurch gekennzeichnet dass**
a) der von der Nukleinsäure-Ausgangssequenz abgeleitete Teil des 5'-Bereiches im Vergleich zur Nukleinsäure-Ausgangssequenz um X - [m x 3] Nukleotide verkürzt ist, wobei m eine ganze Zahl und gleich oder kleiner X/3 ist oder
b) der von der Nukleinsäure-Ausgangssequenz abgeleitete Teil des 3'-Bereiches im Vergleich zur Nukleinsäure-Ausgangssequenz um Y - [n x 3] Nukleotide verkürzt ist, wobei n eine ganze Zahl und gleich oder kleiner Y/3 ist oder
c) der 5'-Bereich und der 3'-Bereich jeweils gemäß (a) und (b) verkürzt sind.

3. Verfahren nach einem der vorhergehenden Ansprüche, wobei die 3' Enden der ersten Teilfragmente mit den 5' Enden der zweiten Teilfragmente mindestens um ungefähr 20 Nukleotide überlappen oder die Nukleinsäuresequenz der 3' Enden der ersten Teilfragmente mit der Nukleinsäuresequenz der 5' Enden der zweiten Teilfragmente zu mindestens ungefähr 70%, bevorzugt mindestens 75% identisch ist, und die ersten Teilfragmente mit den zweiten Teilfragmenten über Fusions-PCR miteinander verbunden werden.

4. Verfahren nach einem der vorhergehenden Ansprüche, wobei die 3' Enden der ersten Teilfragmente und die 5'-Enden der zweiten Teilfragmente einzelsträngige Überhänge aufweisen und die ersten Teilfragmente mit den zweiten Teilfragmenten durch Ligation miteinander verbunden werden,
und optional die einzelsträngigen Überhänge der ersten und zweiten Teilfragmente durch Verdau mit einem Enzym erzeugt werden

5. Verfahren nach einem der vorhergehenden Ansprüche, wobei die Herstellung der ersten Teilfragmente durch folgende Schritte gekennzeichnet ist
a) Bereitstellen einer definierten Nukleinsäure-Ausgangssequenz
b) Bereitstellen von einem ersten Oligonukleotid für die Synthese eines ersten Teilfragments, wobei der 3'-Bereich des ersten Oligonukleotids zu einer definierten Nukleinsäuresequenz im 5'-Bereich der Nukleinsäure-Ausgangssequenz zu mindestens ungefähr 70%, bevorzugt mindestens 75% identisch ist,
c) Bereitstellen von einem zweiten Oligonukleotid wobei der 3'-Bereich des zweiten Oligonukleotids zu einer definierten Nukleinsäuresequenz des konstanten zentralen Bereiches der Nukleinsäure-Ausgangssequenz zu mindestens ungefähr 70%, bevorzugt mindestens 75% identisch ist
d) Inkubation der Nukleinsäure-Ausgangssequenz aus (a) mit dem ersten Oligonukleotid aus (b), dem zweiten Oligonukleotid aus (c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
e) Bereitstellen von einem dritten Oligonukleotid für die Synthese eines zweiten 5'-Teilfragments, wobei der 3'-Bereich des dritten Oligonukleotids zu einer definierten Nukleinsäuresequenz im 5'-Bereich der Nukleinsäure-Ausgangssequenz zu mindestens ungefähr 70%, bevorzugt mindestens 75% identisch ist und sich in mindestens einem, bevorzugt in mindestens drei Nukleotiden von den 3'-Bereichen der ersten Oligonukleotide unterscheidet,
f) Inkubation der Nukleinsäure-Ausgangssequenz aus (a) mit dem dritten Oligonukleotid aus (e), dem zweiten Oligonukleotid aus (c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
g) ggf. Bereitstellen von weiteren Oligonukleotiden für die Synthese von weiteren 5'-Teilfragmenten wobei jeder 3'-Bereich jedes weiteren Oligonukleotids zu einer anderen definierten Nukleinsäuresequenz im 5'-Bereich der Nukleinsäure-Ausgangssequenz zu mindestens ungefähr 70%, bevorzugt mindestens 75% identisch ist und sich in mindestens einem, bevorzugt in mindestens drei Nukleotiden von den 3'-Bereichen der ersten und dritten Oligonukleotide unterscheidet
h) Inkubation der Nukleinsäure-Ausgangssequenz aus (a) mit jeweils einem weiteren Oligonukleotid aus (g), dem zweiten Oligonukleotid aus (c) und einem Polymerasesystem unter Bedingungen, die geeignet sind eine Hybridisierung der homologen 3'-Bereiche der Oligonukleotide an die Nukleinsäure-Ausgangssequenz und deren Verlängerung durch Polymerasekettenreaktion zu ermöglichen
Wiederholung der Schritte (g) und (h), bis alle gewünschten 5'-Teilfragmente synthetisiert sind.

6. Verfahren nach einem der vorhergehenden Ansprüche, wobei die Herstellung der zweiten Teilfragmente durch folgende Schritte gekennzeichnet ist
a) Bereitstellen einer definierten Nukleinsäure-Ausgangssequenz
b) Bereitstellen von einem ersten Oligonukleotid für die Synthese eines ersten 3'-Teilfragments, wobei der 3'-Bereich des ersten Oligonukleotids zu einer definierten Nukleinsäuresequenz im 3'-Bereich der Nukleinsäure-Ausgangssequenz zu mindestens ungefähr 70%, bevorzugt mindestens 75% identisch ist,
c) Bereitstellen von einem zweiten Oligonukleotid wobei der 3'-Bereich des zweiten Oligonukleotids zu einer definierten Nukleinsäuresequenz des konstanten zentralen Bereiches der Nukleinsäure-Ausgangssequenz zu mindestens ungefähr 70%, bevorzugt mindestens 75% identisch ist
d) Inkubation der Nukleinsäure-Ausgangssequenz aus (a) mit dem ersten Oligonukleotid aus (b), dem zweiten Oligonukleotid aus (c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
e) Bereitstellen von einem dritten Oligonukleotid für die Synthese eines zweiten 3'-Teilfragments, wobei der 3'-Bereich des dritten Oligonukleotids zu einer definierten Nukleinsäuresequenz im 3'-Bereich der Nukleinsäure-Ausgangssequenz zu mindestens ungefähr 70%, bevorzugt mindestens 75% identisch ist und sich in mindestens einem, bevorzugt drei Nukleotiden von den 3'-Bereichen der ersten Oligonukleotide unterscheidet,
f) Inkubation der Nukleinsäure-Ausgangssequenz aus (a) mit dem dritten Oligonukleotid aus (e), dem zweiten Oligonukleotid aus (c) und einem Polymerasesystem unter Bedingungen, die geeignet sind eine Hybridisierung der homologen 3'-Bereiche der Oligonukleotide an einzelsträngige Bereiche der Nukleinsäure-Ausgangssequenz und die Verlängerung der hybridisierten Oligonukleotide durch Polymerasekettenreaktion zu ermöglichen
g) ggf. Bereitstellen von weiteren Oligonukleotiden für die Synthese von weiteren 3'-Teilfragmenten wobei jeder 3'-Bereich jedes weiteren Oligonukleotids zu einer anderen definierten Nukleinsäuresequenz im 3'-Bereich der Nukleinsäure-Ausgangssequenz zu mindestens ungefähr 70%, bevorzugt mindestens 75% identisch ist und sich in mindestens einem, bevorzugt mindestens drei Nukleotiden von den 3'-Bereichen der ersten und dritten Oligonukleotide unterscheidet
h) Inkubation der Nukleinsäure-Ausgangssequenz aus (a) mit jeweils einem weiteren Oligonukleotid aus (g), dem zweiten Oligonukleotid aus (c) und einem Polymerasesystem unter Bedingungen, die geeignet sind eine Hybridisierung der homologen 3'-Bereiche der Oligonukleotide an die Nukleinsäure-Ausgangssequenz und deren Verlängerung durch Polymerasekettenreaktion zu ermöglichen
Wiederholung der Schritte (g) und (h), bis alle gewünschten 3'-Teilfragmente synthetisiert sind.

7. Verfahren nach einem der vorhergehenden Ansprüche **gekennzeichnet durch** die folgenden Schritte
a) Herstellung aller ersten Teilfragmente gemäß Anspruch 5 und/oder aller zweiten Teilfragmente gemäß Anspruch 6
b) Vereinigen aller oder einer Auswahl aller ersten Teilfragmente mit allen oder einer Auswahl aller zweiten Teilfragmente und
c) Verbinden der ersten und zweiten Teilfragmente aus der in b) erhaltenen Mischung durch Fusions-PCR oder
d) Inkubation der ersten und zweiten Teilfragmente aus der in b) erhaltenen Mischung mit einem geeigneten Enzym zur Generierung einzelsträngiger Überhänge an den 3' Enden der ersten und den 5' Enden der zweiten Teilfragmente und Verbinden der ersten und zweiten Teilfragmente **durch** Ligation oder Rekombination

8. Verfahren nach einem der vorhergehenden Ansprüche weiterhin umfassend die folgenden Schritte:
a)
(i) Einbringen der Fragment-Bibliothek in einen geeigneten Expressionsvektor,
(ii) Einbringen der Expressionsvektoren enhaltend die Fragment-Bibliothek aus (i) in eukaryontische oder prokaryontische Zellen,
(iii) Kultivieren der Zellen aus (ii) unter Bedingungen, die geeignet sind, eine Expression der Fragment-Bibliothek zu erlauben oder
a)' Inkubation der Fragment-Bibliothek mit einem in vitro Transkriptions-/Translationssystem,
b) gegebenenfalls Isolieren der Polypeptid-Bibliothek aus a) oder a)' und
c) gegebenenfalls Screenen der Polypeptid-Bibliothek aus a), a)' oder b) in einem geeigneten Selektionsverfahren zur Identifikation eines oder mehrerer Kandidaten mit gewünschten Eigenschaften.

## Claims

1. A method for producing a fragment library comprising at least two, preferably at least four, length variants of a nucleic acid starting sequence, **characterized in that**
a) each length variant includes a constant central region and in each case a 5' region and a 3' region of variable length,
b) each length variant is composed at least of a first partial fragment and a second partial fragment, wherein each first partial fragment includes at least one defined 5' partial region of the constant central region, and each second partial fragment includes at least one defined 3' partial region of the constant central region, and the first and second partial fragments have no frameshift mutations with respect to the nucleic acid starting sequence,
c) wherein all first and second partial fragments are joined to one another in one step in such a way that the length variants have all possible combinations of first and second partial fragments,
d) and optionally, it is not possible for a combination of first partial fragments with one another or of second partial fragments with one another to take place.

2. The method according to Claim 1, wherein the 5' region of each length variant is defined by X nucleotides and the 3' region of each length variant is defined by Y nucleotides, X and Y are integers, and the central region is identical in each length variant, **characterized in that**
a) the portion of the 5' region derived from the nucleic acid starting sequence is shortened in comparison to the nucleic acid starting sequence by X - [m x 3] nucleotides, where m is an integer and is less than or equal to X/3, or
b) the portion of the 3' region derived from the nucleic acid starting sequence is shortened in comparison to the nucleic acid starting sequence by Y - [n x 3] nucleotides, where n is an integer and is less than or equal to Y/3, or
c) the 5' region and the 3' region are each shortened according to (a) and (b).

3. The method according to one of the preceding claims, wherein the 3' ends of the first partial fragments overlap with the 5' ends of the second partial fragments at least by approximately 20 nucleotides, or the nucleic acid sequence of the 3' ends of the first partial fragments is identical to the nucleic acid sequence of the 5' ends of the second partial fragments by at least approximately 70%, preferably at least 75%, and the first partial fragments are joined to the second partial fragments by means of fusion PCR.

4. The method according to one of the preceding claims, wherein the 3' ends of the first partial fragments and the 5' ends of the second partial fragments have single-strand overhangs, and the first partial fragments are joined to the second partial fragments by ligation,
and optionally, the single-strand overhangs of the first and second partial fragments are produced by digestion with an enzyme.

5. The method according to one of the preceding claims, wherein the production of the first partial fragments is **characterized by** the following steps:
a) providing a defined nucleic acid starting sequence,
b) providing a first oligonucleotide for the synthesis of a first partial fragment, wherein the 3' region of the first oligonucleotide is identical to a defined nucleic acid sequence in the 5' region of the nucleic acid starting sequence by at least approximately 70%, preferably at least 75%,
c) providing a second oligonucleotide, wherein the 3' region of the second oligonucleotide is identical to a defined nucleic acid sequence of the constant central region of the nucleic acid starting sequence by at least approximately 70%, preferably at least 75%,
d) incubating the nucleic acid starting sequence from (a) with the first oligonucleotide from (b), the second oligonucleotide from (c), and a polymerase system under conditions that are suitable for allowing hybridization of the homologous 3' regions of the oligonucleotides at single-strand regions of the nucleic acid starting sequence and lengthening of the hybridized oligonucleotides by polymerase chain reaction,
e) providing a third oligonucleotide for the synthesis of a second 5' partial fragment, wherein the 3' region of the third oligonucleotide is identical to a defined nucleic acid sequence in the 5' region of the nucleic acid starting sequence by at least approximately 70%, preferably at least 75%, and differs from the 3' regions of the first oligonucleotides in at least one, preferably in at least three, nucleotides,
f) incubating the nucleic acid starting sequence from (a) with the third oligonucleotide from (e), the second oligonucleotide from (c), and a polymerase system under conditions that are suitable for allowing hybridization of the homologous 3' regions of the oligonucleotides to single-strand regions of the nucleic acid starting sequence and lengthening of the hybridized oligonucleotides by polymerase chain reaction,
g) optionally providing further oligonucleotides for the synthesis of further 5' partial fragments, wherein each 3' region of each further oligonucleotide is identical to another defined nucleic acid sequence in the 5' region of the nucleic acid starting sequence by at least approximately 70%, preferably at least 75%, and differs from the 3' regions of the first and third oligonucleotides in at least one, preferably in at least three, nucleotides,
h) incubating the nucleic acid starting sequence from (a) in each case with a further oligonucleotide from (g), the second oligonucleotide from (c), and a polymerase system under conditions that are suitable for allowing hybridization of the homologous 3' regions of the oligonucleotides to the nucleic acid starting sequence and lengthening thereof by polymerase chain reaction,
repeating steps (g) and (h) until all desired 5' partial fragments have been synthesized.

6. The method according to one of the preceding claims, wherein the production of the second partial fragments is **characterized by** the following steps:
a) providing a defined nucleic acid starting sequence,
b) providing a first oligonucleotide for the synthesis of a first 3' partial fragment, wherein the 3' region of the first oligonucleotide is identical to a defined nucleic acid sequence in the 3' region of the nucleic acid starting sequence by at least approximately 70%, preferably at least 75%,
c) providing a second oligonucleotide, wherein the 3' region of the second oligonucleotide is identical to a defined nucleic acid sequence of the constant central region of the nucleic acid starting sequence by at least approximately 70%, preferably at least 75%,
d) incubating the nucleic acid starting sequence from (a) with the first oligonucleotide from (b), the second oligonucleotide from (c), and a polymerase system under conditions that are suitable for allowing hybridization of the homologous 3' regions of the oligonucleotides to single-strand regions of the nucleic acid starting sequence and lengthening of the hybridized oligonucleotides by polymerase chain reaction,
e) providing a third oligonucleotide for the synthesis of a second 3' partial fragment, wherein the 3' region of the third oligonucleotide is identical to a defined nucleic acid sequence in the 3' region of the nucleic acid starting sequence by at least approximately 70%, preferably at least 75%, and differs from the 3' regions of the first oligonucleotides in at least one, preferably three, nucleotides,
f) incubating the nucleic acid starting sequence from (a) with the third oligonucleotide from (e), the second oligonucleotide from (c), and a polymerase system under conditions that are suitable for allowing hybridization of the homologous 3' regions of the oligonucleotides to single-strand regions of the nucleic acid starting sequence and lengthening of the hybridized oligonucleotides by polymerase chain reaction,
g) optionally providing further oligonucleotides for the synthesis of further 3' partial fragments, wherein each 3' region of each further oligonucleotide is identical to another defined nucleic acid sequence in the 3' region of the nucleic acid starting sequence by at least approximately 70%, preferably at least 75%, and differs from the 3' regions of the first and third oligonucleotides in at least one, preferably in at least three, nucleotides,
h) incubating the nucleic acid starting sequence from (a) in each case with a further oligonucleotide from (g), the second oligonucleotide from (c), and a polymerase system under conditions that are suitable for allowing hybridization of the homologous 3' regions of the oligonucleotides to the nucleic acid starting sequence, and lengthening thereof by polymerase chain reaction,
repeating steps (g) and (h) until all desired 3' partial fragments have been synthesized.

7. The method according to one of the preceding claims, **characterized by** the following steps:
a) producing all first partial fragments according to Claim 5 and/or all second partial fragments according to Claim 6,
b) combining all or a selection of all first partial fragments with all or a selection of all second partial fragments, and
c) joining the first and second partial fragments from the mixture obtained in b) by means of fusion PCR, or
d) incubating the first and second partial fragments from the mixture obtained in b) with a suitable enzyme for generating single-strand overhangs at the 3' ends of the first partial fragments and the 5' ends of the second partial fragments, and joining the first and second partial fragments by ligation or recombination.

8. The method according to one of the preceding claims, further comprising the following steps:
a)
(i) introducing the fragment library into a suitable expression vector,
(ii) introducing the expression vectors containing the fragment library from (i) into eukaryotic or prokaryotic cells,
(iii) culturing the cells from (ii) under conditions that are suitable for allowing expression of the fragment library, or
a)' incubating the fragment library with an in vitro transcription/translation system,
b) optionally isolating the polypeptide library from a) or a)', and
c) optionally screening the polypeptide library from a), a)', or b) in a suitable selection method for identifying one or more candidates having desired properties.

## Revendications

1. Procédé de production d'une bibliothèque de fragments comprenant au moins deux, de préférence au moins quatre, variantes de longueur d'une séquence d'acide nucléique de départ, **caractérisé en ce que**
a) chaque variante de longueur comporte une région centrale constante et des régions 5' et 3' de longueur variable,
b) chaque variante de longueur est composée d'au moins un premier sous-fragment et d'un second sous-fragment, chaque premier sous-fragment comportant au moins une sous-région 5' définie de la région centrale constante, et chaque second sous-fragment comportant au moins une sous-région 3' définie de la région centrale constante et les premier et second sous-fragments ne comportant aucune mutations de cadre de lecture par rapport à la séquence d'acide nucléique de départ,
c) tous les premier et second sous-fragments étant reliés entre eux en une seule étape de sorte que les variantes de longueur comportent toutes les combinaisons possibles de premier et second sous-fragments,
d) et, éventuellement, aucune combinaison de premiers sous-fragments entre eux ou de seconds sous-fragments entre eux ne peut être réalisée.

2. Procédé selon la revendication 1, dans lequel la région 5' de chaque variante de longueur est définie par X nucléotides et la région 3' de chaque variante de longueur est définie par Y nucléotides, X et Y sont des nombres entiers et la région centrale est identique dans chaque variante de longueur, **caractérisé en ce que**
a) la partie de la région 5', dérivée de la séquence d'acide nucléique de départ, est réduite de X-[m x 3] nucléotides par rapport à la séquence d'acide nucléique de départ, m étant un nombre entier et étant égal ou inférieur à X/3, ou
b) la partie de la région 3', dérivée de la séquence d'acide nucléique de départ, est réduite de Y-[m x 3] nucléotides par rapport à la séquence d'acide nucléique de départ, m étant un nombre entier et étant égal ou inférieur à X/3, ou
c) la région 5' et la région 3' sont réduite selon (a) et (b) respectivement.

3. Procédé selon l'une quelconque des revendications précédentes, dans lequel les extrémités 3' des premiers sous-fragments chevauchent les extrémités 5' des seconds sous-fragments sur au moins 20 nucléotides environ ou la séquence d'acide nucléique des extrémités 3' des premiers sous-fragments est identique à la séquence d'acide nucléique des extrémités 5' des seconds sous-fragments à au moins 70% environ, de préférence au moins 75%, et les premiers sous-fragments sont reliés aux seconds sous-fragments par fusion en PCR.

4. Procédé selon l'une quelconque des revendications précédentes, dans lequel les extrémités 3' des premiers sous-fragments et les extrémités 5' des seconds sous-fragments comportent des parties saillantes simple brin et les premiers sous-fragments sont reliés aux seconds sous-fragments par ligature,
et éventuellement les parties saillantes simple brin des premiers et seconds sous-fragments sont produites par digestion avec une enzyme.

5. Procédé selon l'une des revendications précédentes, dans lequel la production des premiers sous-fragments est **caractérisée par** les étapes suivantes consistant à
a) produire une séquence d'acide nucléique de départ définie,
b) produire un premier oligonucléotide pour la synthèse d'un premier sous-fragment, la région 3' du premier oligonucléotide étant identique à une séquence d'acide nucléique définie dans la région 5' de la séquence d'acide nucléique de départ à au moins 70% environ, de préférence au moins 75%,
c) produire un deuxième oligonucléotide, la région 3' du second oligonucléotide étant identique à une séquence d'acide nucléique définie de la région centrale constante à au moins 70% environ, de préférence au moins 75%,
d) incuber la séquence d'acide nucléique de départ de (a) avec le premier oligonucléotide de (b), le deuxième oligonucléotide de (c) et un système de polymérase dans des conditions qui sont appropriées pour permettre l'hybridation des régions 3' homologues des oligonucléotides à des régions simple brin de la séquence d'acide nucléique de départ et l'extension des oligonucléotides hybridés par réaction en chaîne par polymérase,
e) produire un troisième oligonucléotide pour la synthèse d'un second sous-fragment 5', la région 3' du troisième oligonucléotide étant identique à une séquence d'acide nucléique définie dans la région 5' de la séquence d'acide nucléique de départ à au moins 70% environ, de préférence au moins 75%, et étant différente des régions 3' du premier oligonucléotide dans au moins un, de préférence dans au moins trois, nucléotides,
f) incuber la séquence d'acide nucléique de départ de (a) avec le troisième oligonucléotide de (e), le deuxième oligonucléotide de (c) et un système de polymérase dans des conditions qui sont appropriées pour permettre l'hybridation des régions 3' homologues des oligonucléotides à des régions simple brin de la séquence d'acide nucléique de départ et l'extension des oligonucléotides hybridés par réaction en chaîne par polymérase,
g) éventuellement produire d'autres oligonucléotides pour la synthèse d'autres sous-fragments 5', chaque région 3' de chaque autre oligonucléotide étant identique à une autre séquence d'acide nucléique définie dans la région 5' de la séquence d'acide nucléique de départ à au moins 70% environ, de préférence au moins 75%, et étant différente des régions 3' des premier et troisième oligonucléotides dans au moins un, de préférence dans au moins trois, nucléotides,
h) incuber la séquence d'acide nucléique de départ de (a) avec une autre oligonucleotide de (g), le deuxième oligonucléotide de (c) et un système de polymérase dans des conditions qui sont appropriées pour permettre l'hybridation des régions 3' homologues 3 des oligonucléotides à la séquence d'acide nucléique de départ et leur extension par réaction en chaîne par polymérase,
répéter les étapes (g) et (h) jusqu'à ce que tous les sous-fragments 5' souhaités soient synthétisés.

6. Procédé selon l'une quelconque des revendications précédentes, dans lequel la production des seconds sous-fragments est **caractérisée par** les étapes suivantes consistant à
a) produire une séquence d'acide nucléique de départ définie,
b) produire un premier oligonucléotide pour la synthèse d'un premier sous-fragment 3', la région 3' du premier oligonucléotide étant identique à une séquence d'acide nucléique définie dans la région 3' de la séquence d'acide nucléique de départ à au moins 70% environ, de préférence au moins 75%,
c) produire un deuxième oligonucléotide, la région 3' du deuxième oligonucléotide étant identique à une séquence d'acide nucléique définie de la région centrale constante de la séquence d'acide nucléique de départ à au moins 70% environ, de préférence au moins 75%,
d) incuber la séquence d'acide nucléique de départ de (a) avec le premier oligonucléotide de (b), le deuxième oligonucléotide de (c) et un système de polymérase dans des conditions qui sont appropriées pour permettre l'hybridation des régions 3' homologues des oligonucléotides à des régions simple brin de la séquence d'acide nucléique de départ et l'extension des oligonucléotides hybridés par réaction en chaîne par polymérase,
e) produire un troisième oligonucléotide pour la synthèse d'un second sous fragment 3', la région 3' du troisième oligonucléotide étant identique à une séquence d'acide nucléique définie dans la région 3' de la séquence d'acide nucléique de départ à au moins 70% environ, de préférence au moins 75%, et étant différente des régions 3' du premier oligonucléotide dans au moins un, de préférence trois, nucléotides,
f) incuber la séquence d'acide nucléique de départ de (a) avec le troisième oligonucléotide de (e), le deuxième oligonucléotide de (c) et un système de polymérase dans des conditions qui sont appropriées pour permettre l'hybridation des régions 3' homologues des oligonucléotides à des régions simple brin de la séquence d'acide nucléique de départ et l'extension des oligonucléotides hybridés par réaction en chaîne par polymérase,
g) éventuellement produire d'autres oligonucléotides pour la synthèse d'autres sous-fragments 3', chaque région 3' de chaque autre oligonucléotide étant identique à une autre séquence d'acide nucléique définie dans la région 3' de la séquence d'acide nucléique de départ à au moins 70% environ, de préférence au moins 75%, et étant différente des régions 3' des premier et troisième oligonucléotides dans au moins un, de préférence au moins trois, nucléotides,
h) incuber la séquence d'acide nucléique de départ de (a) avec une autre oligonucléotide de (g), le deuxième oligonucléotide de (c) et un système de polymérase dans des conditions qui sont appropriées pour permettre l'hybridation des régions 3' homologues des oligonucléotides à la séquence d'acide nucléique de départ et leur extension par réaction en chaîne par polymérase,
répéter les étapes (g) et (h) jusqu'à tous les sous-fragments 3' souhaités soient synthétisés.

7. Procédé selon l'une des revendications précédentes, **caractérisé par** les étapes suivantes consistant à
a) produire tous les premiers sous-fragments selon la revendication 5 et/ou tous les seconds sous-fragments selon la revendication 6,
b) combiner la totalité ou une sélection de tous les premiers sous-fragments avec la totalité ou une sélection de tous les seconds sous-fragments et
c) relier les premiers et seconds sous-fragments du mélange obtenu en b) par fusion en PCR ou
d) incuber les premiers et seconds sous-fragments du mélange obtenu en b) avec une enzyme appropriée pour générer des parties saillantes simple brin aux extrémités 3' des premiers sous-fragments et aux extrémités 5' des seconds sous-fragments et relier les premiers et seconds sous-fragments par ligature ou recombinaison.

8. Procédé selon l'une des revendications précédentes, comprenant en outre les étapes suivantes consistant à :
a)
(i) introduire la bibliothèque de fragments dans un vecteur d'expression approprié,
(ii) introduction les vecteurs d'expression contenant la bibliothèque de fragments de (i) dans des cellules eucaryotes ou procaryotes,
(iii) cultiver les cellules de (ii) dans des conditions qui sont appropriées pour permettre l'expression de la bibliothèque de fragments ou
a)' incuber la bibliothèque de fragments avec un système de transcription/traduction in vitro,
b) éventuellement isoler la bibliothèque de polypeptides de a) ou de a)' et
c) éventuellement cribler la bibliothèque de polypeptides de a), a)' ou b) dans un procédé de sélection approprié pour identifier un ou plusieurs candidats ayant des propriétés souhaitées.


### Sequenzprotokoll

<110> Geneart AG
<120> Verfahren zur Herstellung von Leseraster-korrekten
   Fragment-Bibliotheken
<130> GA016P-DE
<160> 44
<210> 1
   <211> 30
   <212> DNA
   <213> künstliche Sequenz
<220>
   <223> Beschreibung der künstlichen Sequenz: Oligonukleotid
<400> 1
   gcagcatatg agccatattc aacgggaaac 30
<210> 2
   <211> 29
   <212> DNA
   <213> künstliche Sequenz
<220>
   <223> Beschreibung der künstlichen Sequenz: Oligonukleotid
<400> 2
   gcagcatatg caacgggaaa cgtcgaggc 29
<210> 3
   <211> 31
   <212> DNA
   <213> künstliche Sequenz
<220>
   <223> Beschreibung der künstlichen Sequenz: Oligonukleotid
<400> 3
   gcagcatatg acgtcgaggc cgcgattaaa t 31
<210> 4
   <211> 31
   <212> DNA
   <213> künstliche Sequenz
<220>
   <223> Beschreibung der künstlichen Sequenz: Oligonukleotid
<400> 4
   gcagcatatg ccgcgattaa attccaacat g 31
<210> 5
   <211> 30
   <212> DNA
   <213> künstliche Sequenz
<220>
   <223> Beschreibung der künstlichen Sequenz: Oligonukleotid
<400> 5
   gcagcatatg aattccaaca tggatgctga 30
<210> 6
   <211> 31
   <212> DNA
   <213> künstliche Sequenz
<220>
   <223> Beschreibung der künstlichen Sequenz: Oligonukleotid
<400> 6
   gcagcatatg atggatgctg atttatatgg g 31
<210> 7
   <211> 32
   <212> DNA
   <213> künstliche Sequenz
<220>
   <223> Beschreibung der künstlichen Sequenz: Oligonukleotid
<400> 7
   gcagcatatg gatttatatg ggtataaatg gg 32
<210> 8
   <211> 29
   <212> DNA
   <213> künstliche Sequenz
<220>
   <223> Beschreibung der künstlichen Sequenz: Oligonukleotid
<400> 8
   gcagcatatg gggtataaat gggctcgcg 29
<210> 9
   <211> 29
   <212> DNA
   <213> künstliche Sequenz
<220>
   <223> Beschreibung der künstlichen Sequenz: Oligonukleotid
<400> 9
   gcagcatatg tgggctcgcg ataatgtcg 29
<210> 10
   <211> 29
   <212> DNA
   <213> künstliche Sequenz
<220>
   <223> Beschreibung der künstlichen Sequenz: Oligonukleotid
<400> 10
   gcagcatatg gataatgtcg ggcaatcag 29
<210> 11
   <211> 31
   <212> DNA
   <213> künstliche Sequenz
<220>
   <223> Beschreibung der künstlichen Sequenz: Oligonukleotid
<400> 11
   gcagcatatg gggcaatcag gtgcgacaat c 31
<210> 12
   <211> 31
   <212> DNA
   <213> künstliche Sequenz
<220>
   <223> Beschreibung der künstlichen Sequenz: Oligonukleotid
<400> 12
   gcagcatatg ggtgcgacaa tctatcgctt g 31
<210> 13
   <211> 31
   <212> DNA
   <213> künstliche Sequenz
<220>
   <223> Beschreibung der künstlichen Sequenz: Oligonukleotid
<400> 13
   gcagcatatg atctatcgct tgtatgggaa g 31
<210> 14
   <211> 29
   <212> DNA
   <213> künstliche Sequenz
<220>
   <223> Beschreibung der künstlichen Sequenz: Oligonukleotid
<400> 14
   gcagcatatg ttgtatggga agcccgatg 29
<210> 15
   <211> 28
   <212> DNA
   <213> künstliche Sequenz
<220>
   <223> Beschreibung der künstlichen Sequenz: Oligonukleotid
<400> 15
   gcagcatatg aagcccgatg cgccagag 28
<210> 16
   <211> 28
   <212> DNA
   <213> künstliche Sequenz
<220>
   <223> Beschreibung der künstlichen Sequenz: Oligonukleotid
<400> 16
   gcagcatatg gcgccagagt tgtttctg 28
<210> 17
   <211> 31
   <212> DNA
   <213> künstliche Sequenz
<220>
   <223> Beschreibung der künstlichen Sequenz: Oligonukleotid
<400> 17
   gcagcatatg gttgtttctg aaacatggca a 31
<210> 18
   <211> 29
   <212> DNA
   <213> künstliche Sequenz
<220>
   <223> Beschreibung der künstlichen Sequenz: Oligonukleotid
<400> 18
   gcagcatatg gaaacatggc aaaggtagc 29
<210> 19
   <211> 30
   <212> DNA
   <213> künstliche Sequenz
<220>
   <223> Beschreibung der künstlichen Sequenz: Oligonukleotid
<400> 19
   gcagcatatg caaaggtagc gttgccaatg 30
<210> 20
   <211> 30
   <212> DNA
   <213> künstliche Sequenz
<220>
   <223> Beschreibung der künstlichen Sequenz: Oligonukleotid
<400> 20
   gcagcatatg cgttgccaat gatgttacag 30
<210> 21
   <211> 32
   <212> DNA
   <213> künstliche Sequenz
<220>
   <223> Beschreibung der künstlichen Sequenz: Oligonukleotid
<400> 21
   cgtgctcgag ttagaaaaac tcatcgagca tc 32
<210> 22
   <211> 34
   <212> DNA
   <213> künstliche Sequenz
<220>
   <223> Beschreibung der künstlichen Sequenz: Oligonukleotid
<400> 22
   cgtgctcgag ttaatcgagc atcaaatgaa actg 34
<210> 23
   <211> 35
   <212> DNA
   <213> künstliche Sequenz
<220>
   <223> Beschreibung der künstlichen Sequenz: Oligonukleotid
<400> 23
   cgtgctcgag ttacaaatga aactgcaatt tattc 35
<210> 24
   <211> 34
   <212> DNA
   <213> künstliche Sequenz
<220>
   <223> Beschreibung der künstlichen Sequenz: Oligonukleotid
<400> 24
   cgtgctcgag ttactgcaat ttattcatat cagg 34
<210> 25
   <211> 36
   <212> DNA
   <213> künstliche Sequenz
<220>
   <223> Beschreibung der künstlichen Sequenz: Oligonukleotid
<400> 25
   cgtgctcgag ttaattcata tcaggattat caatac 36
<210> 26
   <211> 37
   <212> DNA
   <213> künstliche Sequenz
<220>
   <223> Beschreibung der künstlichen Sequenz: Oligonukleotid
<400> 26
   cgtgctcgag ttaaggatta tcaataccat atttttg 37
<210> 27
   <211> 36
   <212> DNA
   <213> künstliche Sequenz
<220>
   <223> Beschreibung der künstlichen Sequenz: Oligonukleotid
<400> 27
   cgtgctcgag ttaaatacca tatttttgaa aaagcc 36
<210> 28
   <211> 34
   <212> DNA
   <213> künstliche Sequenz
<220>
   <223> Beschreibung der künstlichen Sequenz: Oligonukleotid
<400> 28
   cgtgctcgag ttatttttga aaaagccgtt tctg 34
<210> 29
   <211> 34
   <212> DNA
   <213> künstliche Sequenz
<220>
   <223> Beschreibung der künstlichen Sequenz: Oligonukleotid
<400> 29
   cgtgctcgag ttaaagccgt ttctgtaatg aagg 34
<210> 30
   <211> 34
   <212> DNA
   <213> künstliche Sequenz
<220>
   <223> Beschreibung der künstlichen Sequenz: Oligonukleotid
<400> 30
   cgtgctcgag ttactgtaat gaaggagaaa actc 34
<210> 31
   <211> 32
   <212> DNA
   <213> künstliche Sequenz
<220>
   <223> Beschreibung der künstlichen Sequenz: Oligonukleotid
<400> 31
   cgtgctcgag ttaaggagaa aactcaccga gg 32
<210> 32
   <211> 34
   <212> DNA
   <213> künstliche Sequenz
<220>
   <223> Beschreibung der künstlichen Sequenz: Oligonukleotid
<400> 32
   cgtgctcgag ttagcagttc cataggatgg caag 34
<210> 33
   <211> 34
   <212> DNA
   <213> künstliche Sequenz
<220>
   <223> Beschreibung der künstlichen Sequenz: Oligonukleotid
<400> 33
   cgtgctcgag ttagcagttc cataggatgg caag 34
<210> 34
   <211> 32
   <212> DNA
   <213> künstliche Sequenz
<220>
   <223> Beschreibung der künstlichen Sequenz: Oligonukleotid
<400> 34
   cgtgctcgag ttataggatg gcaagatcct gg 32
<210> 35
   <211> 33
   <212> DNA
   <213> künstliche Sequenz
<220>
   <223> Beschreibung der künstlichen Sequenz: Oligonukleotid
<400> 35
   cgtgctcgag ttaaagatcc tggtatcggt ctg 33
<210> 36
   <211> 32
   <212> DNA
   <213> künstliche Sequenz
<220>
   <223> Beschreibung der künstlichen Sequenz: Oligonukleotid
<400> 36
   cgtgctcgag ttagtatcgg tctgcgattc cg 32
<210> 37
   <211> 31
   <212> DNA
   <213> künstliche Sequenz
<220>
   <223> Beschreibung der künstlichen Sequenz: Oligonukleotid
<400> 37
   cgtgctcgag ttatgcgatt ccgactcgtc c 31
<210> 38
   <211> 33
   <212> DNA
   <213> künstliche Sequenz
<220>
   <223> Beschreibung der künstlichen Sequenz: Oligonukleotid
<400> 38
   cgtgctcgag ttagactcgt ccaacatcaa tac 33
<210> 39
   <211> 38
   <212> DNA
   <213> künstliche Sequenz
<220>
   <223> Beschreibung der künstlichen Sequenz: Oligonukleotid
<400> 39
   cgtgctcgag ttaaacatca atacaaccta ttaatttc 38
<210> 40
   <211> 34
   <212> DNA
   <213> künstliche Sequenz
<220>
   <223> Beschreibung der künstlichen Sequenz: Oligonukleotid
<400> 40
   cgtgctcgag ttaacaacct attaatttcc cctc 34
<210> 41
   <211> 35
   <212> DNA
   <213> künstliche Sequenz
<220>
   <223> Beschreibung der künstlichen Sequenz: Oligonukleotid
<400> 41
   gcacggtacc gaagacaccg gcgcaggaac actgc 35
<210> 42
   <211> 36
   <212> DNA
   <213> künstliche Sequenz
<220>
   <223> Beschreibung der künstlichen Sequenz: Oligonukleotid
<400> 42
   gcacgagctc gaagacgcgc cggttgcatt cgattc 36
<210> 43
   <211> 810
   <212> DNA
   <213> künstliche Sequenz
<220>
   <223> Beschreibung der künstlichen Sequenz: synthetisches Gen
<220>
   <223> nptII
<400> 43
   atgagccata ttcaacggga aacgtcgagg ccgcgattaa attccaacat ggatgctgat   60
   ttatatgggt ataaatgggc tcgcgataat gtcgggcaat caggtgcgac aatctatcgc  120
   ttgtatggga agcccgatgc gccagagttg tttctgaaac atggcaaagg tagcgttgcc  180
   aatgatgtta cagatgagat ggtcagacta aactggctga cggaatttat gcctcttccg  240
   accatcaagc attttatccg tactcctgat gatgcatggt tactcaccac tgcgatcccc  300
   ggaaaaacag cattccaggt attagaagaa tatcctgatt caggtgaaaa tattgttgat  360
   gcgctggcag tgttcctgcg ccggttgcat tcgattcctg tttgtaattg tccttttaac  420
   agcgatcgcg tatttcgtct cgctcaggcg caatcacgaa tgaataacgg tttggttgat  480
   gcgagtgatt ttgatgacga gcgtaatggc tggcctgttg aacaagtctg gaaagaaatg  540
   cataaacttt tgccattctc accggattca gtcgtcactc atggtgattt ctcacttgat  600
   aaccttattt ttgacgaggg gaaattaata ggttgtattg atgttggacg agtcggaatc  660
   gcagaccgat accaggatct tgccatccta tggaactgcc tcggtgagtt ttctccttca  720
   ttacagaaac ggctttttca aaaatatggt attgataatc ctgatatgaa taaattgcag  780
   tttcatttga tgctcgatga gtttttctaa                                   810
<210> 44
   <211> 269
   <212> PRT
   <213> künstliche Sequenz
<220>
   <223> Beschreibung der künstlichen Sequenz: Aminoglycosid-3'-Phosphotransferase des Typs II
<400> 44
   Met Ser His Ile Gln Arg Glu Thr Ser Arg Pro Arg Leu Asn Ser Asn
    1               5                  10                  15
   Met Asp Ala Asp Leu Tyr Gly Tyr Lys Trp Ala Arg Asp Asn Val Gly
               20                  25                  30
   Gln Ser Gly Ala Thr Ile Tyr Arg Leu Tyr Gly Lys Pro Asp Ala Pro
           35                  40                  45
   Glu Leu Phe Leu Lys His Gly Lys Gly Ser Val Ala Asn Asp Val Thr
       50                  55                  60
   Asp Glu Met Val Arg Leu Asn Trp Leu Thr Glu Phe Met Pro Leu Pro
   65                  70                  75                  80
   Thr Ile Lys His Phe Ile Arg Thr Pro Asp Asp Ala Trp Leu Leu Thr
                   85                  90                  95
   Thr Ala Ile Pro Gly Lys Thr Ala Phe Gln Val Leu Glu Glu Tyr Pro
               100                 105                 110
   Asp Ser Gly Glu Asn Ile Val Asp Ala Leu Ala Val Phe Leu Arg Arg
           115                 120                 125
   Leu His Ser Ile Pro Val Cys Asn Cys Pro Phe Asn Ser Asp Arg Val
       130                 135                 140
   Phe Arg Leu Ala Gln Ala Gln Ser Arg Met Asn Asn Gly Leu Val Asp
   145                 150                 155                 160
   Ala Ser Asp Phe Asp Asp Glu Arg Asn Gly Trp Pro Val Glu Gln Val
                   165                 170                 175
   Trp Lys Glu Met His Lys Leu Leu Pro Phe Ser Pro Asp Ser Val Val
               180                 185                 190
   Thr His Gly Asp Phe Ser Leu Asp Asn Leu Ile Phe Asp Glu Gly Lys
           195                 200                 205
   Leu Ile Gly Cys Ile Asp Val Gly Arg Val Gly Ile Ala Asp Arg Tyr
       210                 215                 220
   Gln Asp Leu Ala Ile Leu Trp Asn Cys Leu Gly Glu Phe Ser Pro Ser
   225                 230                 235                 240
   Leu Gln Lys Arg Leu Phe Gln Lys Tyr Gly Ile Asp Asn Pro Asp Met
                   245                 250                 255
   Asn Lys Leu Gln Phe His Leu Met Leu Asp Glu Phe Phe